# EUROPEAN PATENT APPLICATION

(11) **EP 2 365 091 A1**
(43) Date of publication of application: **14.09.2011**
(21) Application number: 10156404.5
(22) Date of filing: 12.03.2010
(51) Int. Cl.: C12Q 1/37, G01N 33/569, A61K 38/00, A61K 38/03, A61K 38/55

(54) **Method of screening for HtrA protease inhibitors useful for prophylaxis and therapy of a bacterial infection**

(71) Applicant: Paul-Ehrlich-Institut, 63225 Langen (DE); Johann Wolfgang Goethe-Universität Frankfurt am Main, 60325 Frankfurt am Main (DE)
(72) Inventor: The designation of the inventor has not yet been filed
(74) Representative: Schüssler, Andrea

(57) **Abstract**

Described are a method of screening for an agent capable of inhibiting protease HtrA which is useful in the prophylaxis or treatment of a bacterial infection, e.g., a *Helicobacter* pyloris infection as well as HtrA inhibitors and their uses.

## Description

The present invention relates to a method of screening for a therapeutic agent capable of inhibiting protease HtrA which is useful in the prophylaxis or treatment of a bacterial infection, e.g., a *Helicobacter pyloris* infection, as well as HtrA inhibitors and their uses.

Gastric cancer is the second leading cause of cancer-related mortality worldwide. Gastric cancer is generally associated with poor prognosis and a relatively high mortality rate. Although early gastric cancer is curable with surgery, often local or distant tumor recurrence occurs despite complete tumor resection. Moreover, a large proportion of patients in Western countries have their cancer only detected in a late stage. Late-stage diagnosis and the lack of effective systemic therapies result in a high mortality rate.

It is generally thought that gastric cancer develops via progression from chronic gastritis to atrophic gastritis, intestinal metaplasia, dysplasia and subsequently to cancer. The most common trigger of this cascade is a *Helicobacter pylori* infection. In the Western European population, the prevalence of *H. pylori* infection is approximately 30-40%, while in Asia the prevalence is almost 60%. *H. pylori* infection is thought to be acquired during infancy or childhood. The human pathogen *Helicobacter pylori* (*Hp*) exclusively colonizes the gastric epithelium niche in the stomach, where it disrupts mucosal integrity and induces inflammatory responses, leading to gastritis, ulceration and neoplastic degenerative MALT (mucosa-associated lymphoid tissue) lymphoma or gastric cancer (Peek, Jr. and Crabtree, 2006; Fujikawa et al., 2003; Matsumoto et al., 2007). The bacteria colonize the gastric mucosa over a long latency period before causing a malignancy. Infection is thought to increase the risk for the development of gastric cancer twofold.

However, although 50% of the world population is infected by *H. pylori* during their lifetime, only about 5% develop gastric cancer. Individual differences in response to infection caused by gene polymorphisms may determine whether a host develops gastric cancer. The risk of developing gastric cancer is thought to be determined by the level of inflammation that occurs due to *H. pylori* infection and subsequent gastric acid secretion. Although a large number of patients will be unaffected by the infection, others may develop gastritis and increased acid secretion, leading to duodenal ulcer. Patients who suffer under chronic gastritis have a higher risk to develop gastric cancer.

Healthy epithelia form a first barrier against pathogens (e.g. *H. pylori),* which depends on the integrity of apical-basal polarity and adhesive complexes, including apical tight junctions (TJs) and basolateral E-Cadherin-based adherence junctions (AJs) (Niessen, 2007). E-Cadherin-mediated intercellular adhesions connect via homophilic interactions between E-Cadherin ectodomains on the surface of neighboring epithelial cells, while the cytoplasmic E-Cadherin domain binds either to β-Catenin or plakoglobin (γ-Catenin) and p120 catenin (p120^{ctn}). These interactions control Cadherin turnover and stabilize the adhesion complex (Niessen, 2007). Functionally, the intact E-Cadherin gene is considered to be an important tumor suppressor gene, since disintegration of AJs through loss of expression, loss-of-function mutations, or proteolytic E-Cadherin truncation is associated with enhanced tumor progression (Chan, 2006).

Disruption of the *Hp*-colonized epithelial layer involves injection of the bacterial effector protein CagA via a type IV secretion system (T4SS) into the host cell cytoplasm, where it can interact with proteins of tight and adherence junction complexes to abrogate epithelial polarity and cell adhesion (Amieva et al., 2003; Murata-Kamiya et al., 2007; Saadat et al., 2007; Suzuki et al., 2005). However, accumulating data indicate that *Hp* can also disrupt intercellular adhesions independently of CagA. This as yet unknown mechanism points to *Hp* pathogenesis being a multistep process (Wessler and Backert, 2008). The main argument for a multistep process was the finding that the T4SS requires the pilus surface protein CagL and β1-integrin as receptor prior to CagA injection (Kwok et al., 2007). Importantly, it remained entirely unknown how *Hp* disintegrates the polarized intestinal epithelium exhibiting intact intercellular adhesions to target basolateral expressed β1-integrin.

Once *H.pylori* is detected in patients with a peptic ulcer, the normal procedure is to eradicate it and allow the ulcer to heal. The standard first-line therapy is a one week *triple therapy* consisting of a proton pump inhibitor such as omeprazole and the antibiotics clarithromycin and amoxicillin. Variations of the triple therapy have been developed over the years, such as using a different proton pump inhibitor, as with pantoprazole or rabeprazole, or replacing amoxicillin with metronidazole for people who are allergic to penicillin. Such a therapy has revolutionized the treatment of peptic ulcers and has made a cure to the disease possible. Previously the only option was symptom control using antacids, H₂-antagonists or proton pump inhibitors alone. However, an increasing number of infected individuals are found to harbour antibiotic-resistant bacteria. This results in initial treatment failure and requires additional rounds of antibiotic therapy or alternative strategies such as a quadruple therapy, which adds a bismuth colloid. Moreover, up to 50 percent of patients have side effects while taking *H. pylori* treatment, e.g., diarrhoea and stomach cramps, and up to 20 percent of patients with *Helicobacter pylori* infection are not cured after completing their first course of treatment.

Thus, the technical problem underlying the present invention is to provide alternative treatment regimens for *H. pylori infections.*

The solution to said technical problem is achieved by providing the embodiments characterized in the claims.

The human pathogen *Helicobacter pylori* (*Hp*) injects the virulence factor CagA into the host cytoplasm where it activates cancer-associated signal transduction. Previously, it could be shown that *Hp* interacts with β1-integrin as receptor for CagA injection. However, it remained unclear how *Hp* can target basolateral expressed β1-integrins when lateral cell adhesions are still intact in polarized epithelial cells. During the experiments leading to the present invention HtrA was identified as a novel proteolytic virulence factor secreted by *Hp* which cleaves the ectodomain of E-Cadherin that exhibits crucial functions in intercellular adhesion and tumour suppression. HtrA-mediated cleavage of E-Cadherin disrupts cell adhesions and induces disintegration of the epithelial barrier function. After E-Cadherin cleavage, *Hp* enters the intercellular space allowing CagA injection across β1-integrins. An assay for screening for HtrA protease inhibitors useful for prophylaxis or therapy of a bacterial infection could be developed which is based on the blocked cleavage of E-Cadherin by HtrA. Altogether, HtrA-mediated E-Cadherin cleavage is a novel mechanism allowing persistent *Hp* colonization and pathogenicity. The results give an explanation how *Hp* disrupts the epithelial barrier to invade the intercellular space of epithelial cells where it can induce pathogenesis. These observations suggest that *Hp-*mediated E-Cadherin ectodomain shedding is an important step in the pathogenesis of inflammatory responses or neoplastic transformation. Pharmacological inhibition of bacterial HtrA might serve as an effective alternative strategy to combat multiple bacterial infections. As a first step, a small molecule inhibitor (HHI) was designed which profoundly blocks HtrA activity and CagA injection and is a potent (lead) structure for pharmacological inhibition of bacterial HtrA.

### Brief description of the drawings

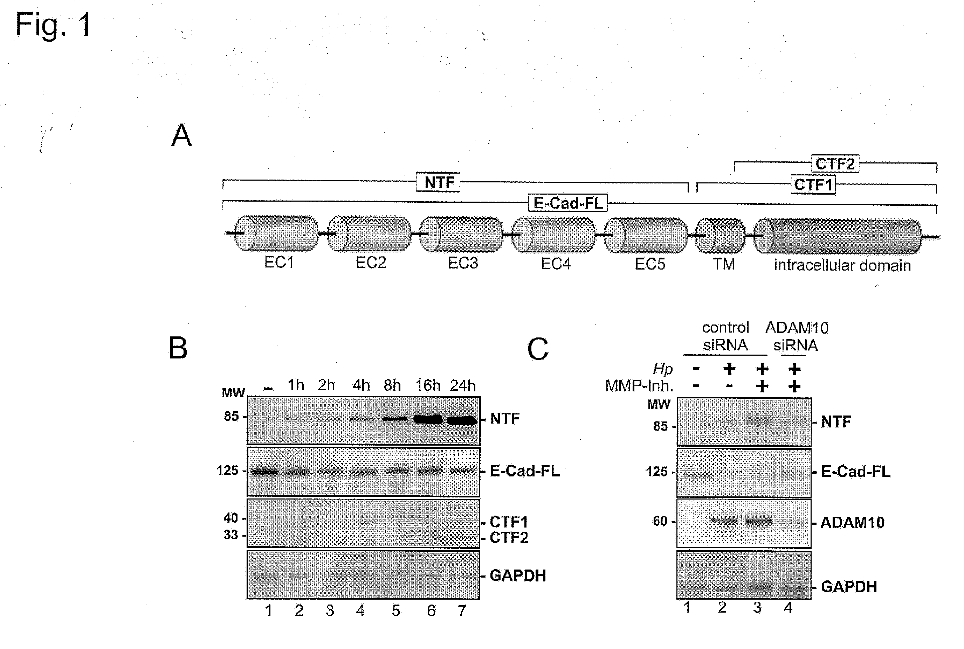

Figure 1: *Hp* induces ectopic E-Cadherin shedding independently of MMPs or ADAM10
   (**A**) Domain structure and cleavage fragments of E-Cadherin. E-Cadherin has five extracellular domains (EC1-EC5), a transmembrane (TM) and an intracellular domain. Cleavage generates the extracellular N-terminal fragment (NTF), and two C-terminal fragments (CTF1, CTF2) containing the cytoplasmic domain of E-Cadherin.
   (**B**) MKN-28 cells were infected with *Hp* for the indicated time periods and increasing amounts of the soluble 85 kDa E-Cadherin fragment (NTF) were detected in the supernatant of infected host cells by Western blots using the E-Cadherin ectodomain antibody. Loss of full length E-Cadherin (E-Cad-FL) in whole cell lysates was confirmed using an antibody detecting intracellular CTF1 and CTF2. GAPDH is shown as a control.
   (C) MKN-28 cells were preincubated with 150 nM MMP inhibitor II (5x IC50) or treated with siRNA targeting ADAM10. As indicated, cells were infected with *Hp*. Soluble NTF in supernatants and E-Cad-FL in whole cell lysates were detected. Down-regulation of mature ADAM10 (60 kDa) and GAPDH were shown as controls.

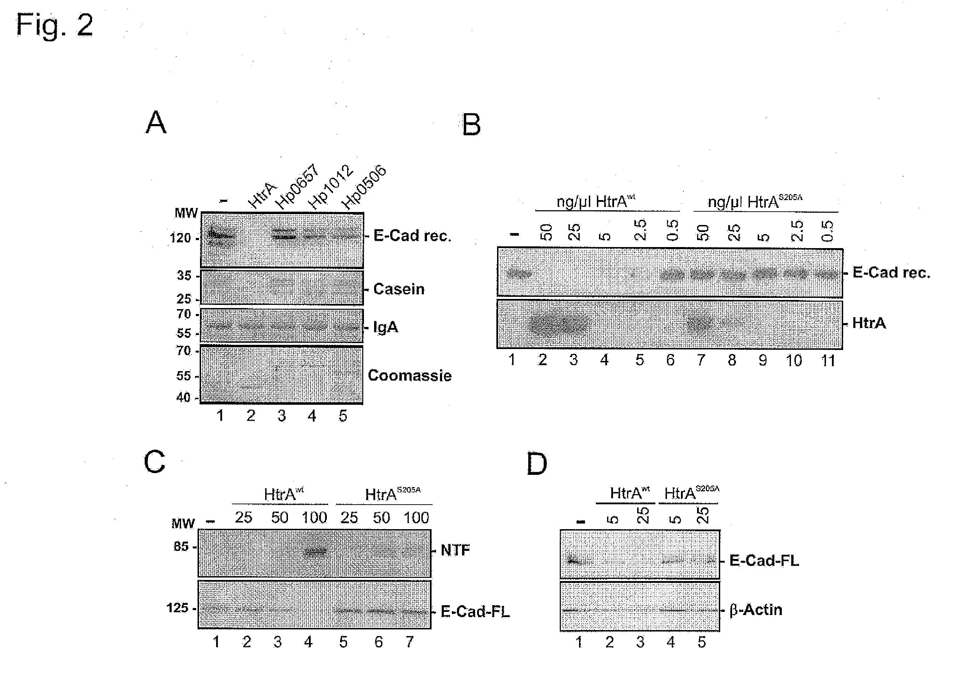

Figure 2: *Hp*-secreted HtrA cleaves E-Cadherin on the cell surface
   (**A**) 100 ng recombinant E-Cadherin (E-Cad rec.) were incubated with 25 ng/µl of the putative *Hp* proteases HtrA, Hp0657, Hp1012 or Hp0506 for 16 h and detected by Western blot. Casein or IgA were used as a positive and negative control, respectively and detected by Coomassie staining.
   (**B**) 100 ng of recombinant E-Cadherin were incubated with decreasing amounts (50-0.5 ng/µl) of purified HtrA^{wt} or inactive HtrA^{S205A} for 16 h. E-Cadherin (E-Cad rec.) and HtrA were detected by Western blots.
   (**C**) Ectopic E-Cadherin (NTF) cleavage and loss of full length E-Cadherin (E-Cad-FL) in confluent MKN-28 cells were investigated after 6 h incubation with 25, 50 and 100 ng/µl of HtrA^{wt} or HtrA^{S205A}.
   (**D**) Detached MKN-28 cells were incubated in culture medium for 3 h for re-expression of E-Cad-FL followed by treatment with indicated concentrations of HtrA^{wt} or HtrA^{S205A} for 1 h. Loss of E-Cad-FL and equal β-actin expression were detected by Western blot.

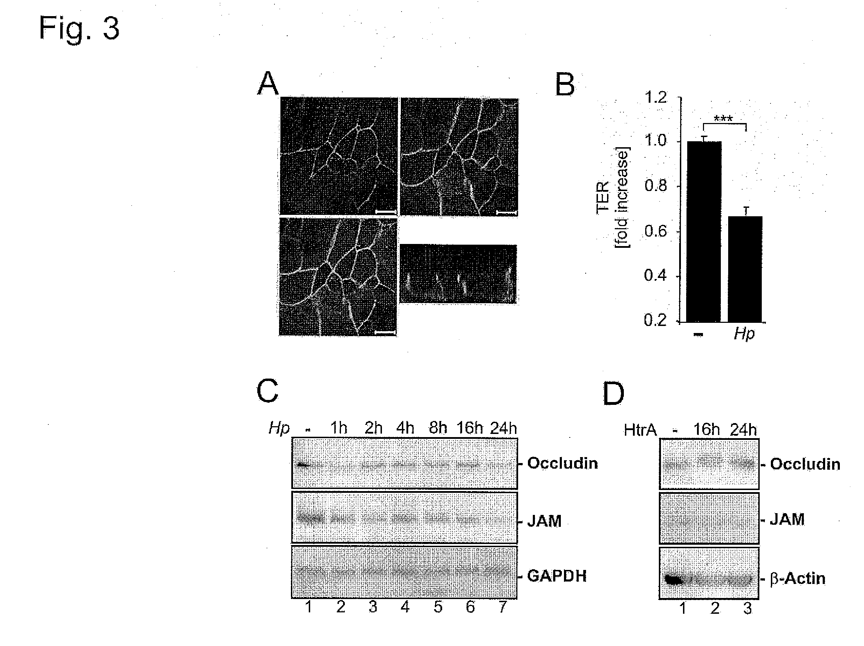

Figure 3: *Hp* disrupts tight junctions independently of HtrA
   (**A**) Confluent MKN-28 cells were stained for ZO-1 (red) and E-Cadherin (green) followed by confocal microscopy along the xy-and xz-axis.
   (**B**) Transepithelial electrical resistance (TER) of MKN-28 cell monolayers was measured for 48 h. Infection with *Hp* decreased TER compared to non-treated (-) MKN-28 cells (*** *p* = 0.00057).
   (**C**) Expression of the TJ proteins Occludin and JAM in response to *Hp* infection was analyzed by Western blot using specific antibodies. GAPDH was shown as a loading control.
   (**D**) MKN-28 cells were stimulated with 100 ng/µl HtrA for 16 h and 24 h. Amounts of Occludin, JAM and β-actin were detected by Western blot.

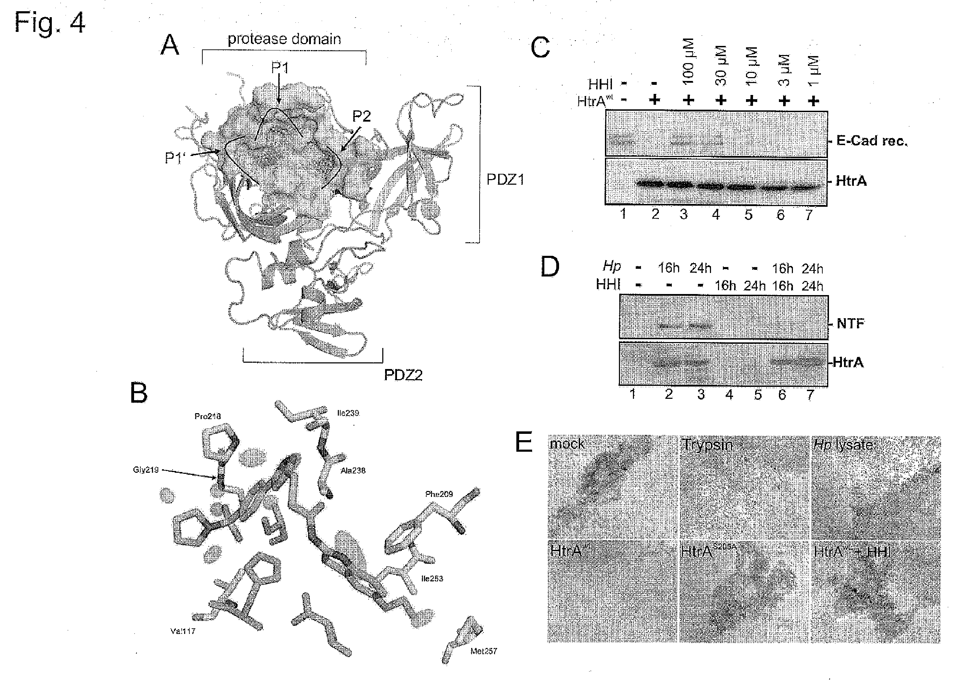

Figure 4: Design of functional small molecule inhibitors targeting HtrA
   (**A**) Ribbon model of the HtrA homolog DegP from *Escherichia coli* with annotated domain organization. Blue spheres indicate the predicted ligand binding sites, shaded by their predicted buried state. The solvent accessible surface is shown; the surface patch contributed by the active site serine 205 is highlighted in red.
   (**B**) Inhibitor HHI docked into the active site of HtrA with potential pharmacophoric interaction sites used for virtual screening (green: lipophilic, red: hydrogen-bond donor). The residues of the catalytic triad are colored in dark grey.
   (**C**) 100 ng of recombinant E-Cadherin incubated *in vitro* with 100 ng/µl of purified HtrA and indicated concentrations of HHI for 16 h. E-Cadherin (E-Cad rec.) and HtrA were detected by Western blots.
   (**D**) MKN-28 cells were infected with *Hp* for 16 and 24 h. Where indicated, cells were co-treated with 100 µM HHI. Soluble extracellular E-Cadherin fragments (NTF) and secreted HtrA were detected in the cell supernatants by Western blots.
   (**E**) E-Cadherin-mediated cell aggregation was monitored with untreated (mock) or trypsin-treated MKN-28 cells. Cells were also incubated with *Hp* lysate at an MOI of 100, or 50 ng/µl of HtrA^{wt}, HtrA^{S205A} or HtrA^{wt} plus 100 µM HHI. Cell aggregates were observed under the microscope.

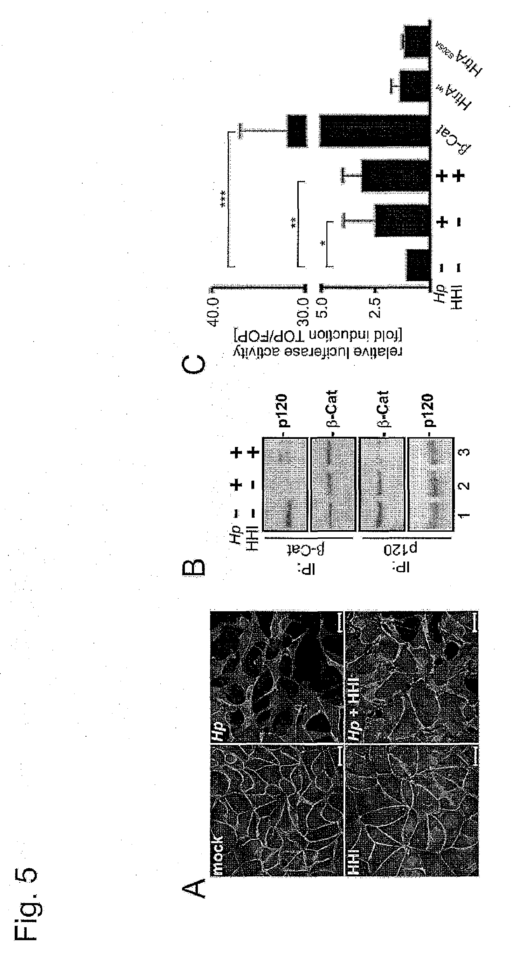

Figure 5: β-Catenin signaling occurs independently of HtrA-mediated E-Cadherin cleavage
   (**A**) Confluent MCF-7 cells were untreated (mock) or infected with *Hp* for 16 h. As indicated, cells were co-treated with 100 µM HHI. E-Cadherin (green) was stained using an antibody detecting the intracellular domain. Nuclei (blue) were stained using DAPI. Scale bar, 20 µm.
   (**B**) MKN-28 cells were infected with *Hp* for 6 h in the presence of 100 µM HHI where indicated. β-Catenin or p120^{ctn} were immunoprecipitated (IP) and interacting p120^{ctn} or β-Catenin were detected by Western blot, respectively.
   (**C**) MKN-28 cells were transiently transfected with 100 ng TOPflash or FOPflash, and with a construct encoding constitutively active β-Catenin (β-Cat^{S33A}) as indicated. Transfected cells were then infected with *Hp* or left untreated (mock) for 24 h. Where indicated, cells were treated with 50 µM HHI or 50 ng/µl HtrA^{wt} or HtrA^{S205A}. The data represent the ratio of TOP/FOP ± S.D. calculated from three independent experiments as x-fold induction compared to the activity observed of unstimulated cells.

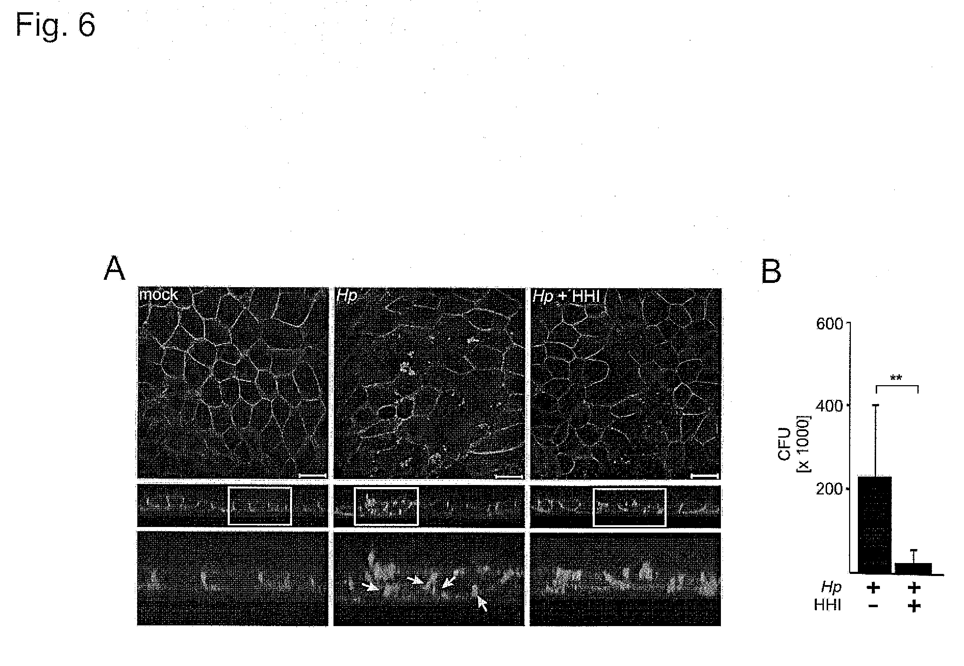

Figure 6: *Hp* HtrA disrupts intercellular adhesions to enter the intercellular space
   (**A**) Confocal microscopy of the extracellular domain of E-Cadherin (red) at the membranes of confluent MKN-28 cells along the *xy*- and *xz*-axis demonstrated basolateral E-Cadherin membrane localization. Scanning along the xz-axis revealed *Hp* (green) in the intercellular space between MKN-28 cells (white arrows) 48 h post-infection. This was inhibited by the addition of 100 µM HHI. Scale bar, 20 µm.
   (**B**) *Hp* translocating across confluent epithelial monolayers was quantified by growing 2.5 x 10⁶ MKN-28 cells on filters, untreated or treated with 100 µM HHI prior to infection with 2.5 x 10⁸ *Hp* for 48 h. Bacteria migrating across the monolayer on the filter were harvested, cultured and colony forming units (CFU) were counted. Incubation of cells with HHI had a significant effect on preventing *Hp* translocation through the intercellular space (** p = 0.01).

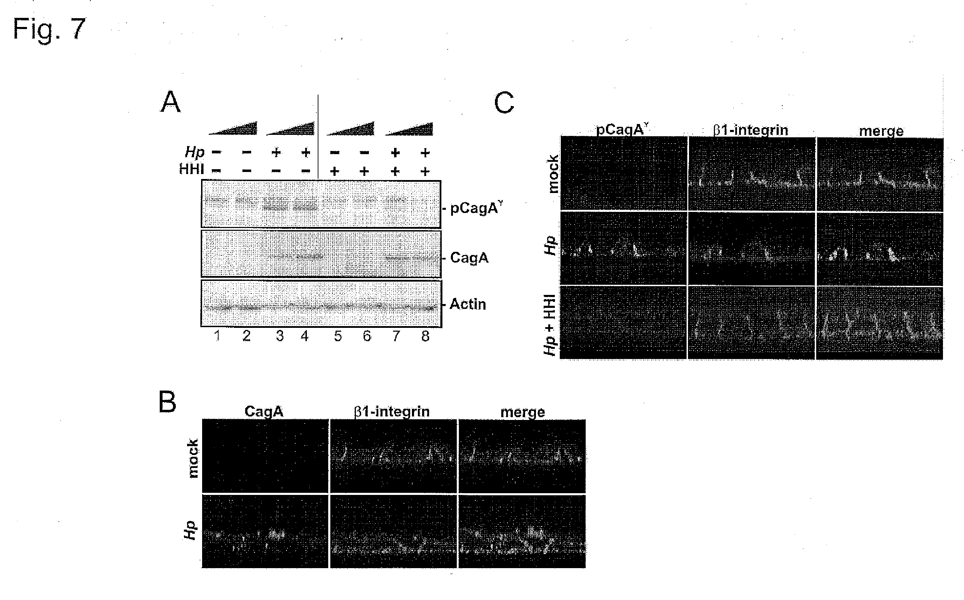

Figure 7: HtrA-mediated E-Cadherin cleavage allows CagA injection into confluent epithelial cells
   (**A**) Phosphorylation of CagA (pCag^{Y}), injected CagA and secreted HtrA were examined in polarized MKN-28 cells after infection with *Hp* for 16 h and 24 h. Preincubation of cells with 100 µM HHI inhibited *Hp*-injected CagA. β-actin is shown as a control.
   (**B**) MCF-7 cells were infected with *Hp* or left untreated (mock) for 24 h. Localization of CagA (green) and basolateral expressed β1-integrin (red) was analyzed by confocal microscopy along the *xz*-axis.
   (**C**) Detection of pCagA^{Y} (green) revealed basolateral co-localization with β1-integrin (red) in *Hp*-infected cells, which was prevented by preincubation with 100 µM HHI.

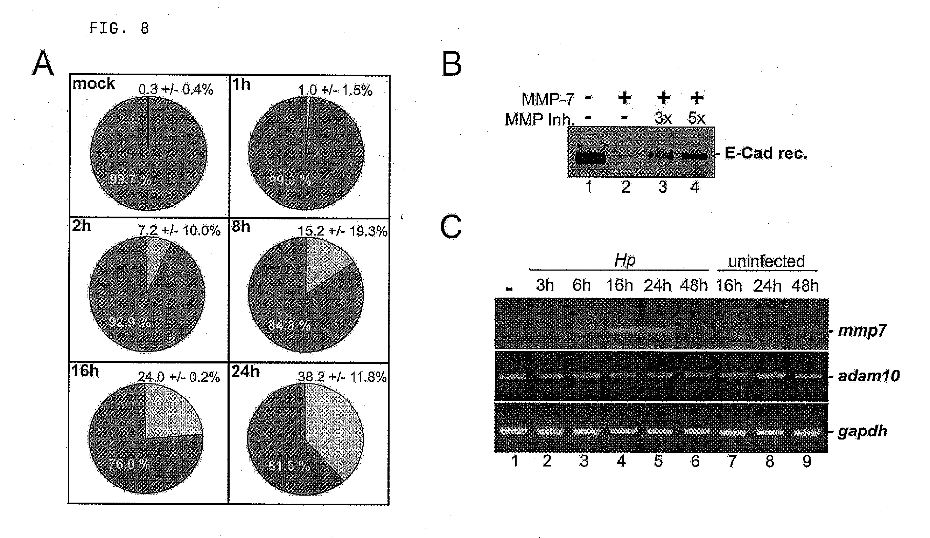

Figure 8: E-Cadherin cleavage is independent of cellular host proteases
   (**A**) Increase in the soluble E-Cadherin fragment NTF (light blue) and loss of full length E-Cadherin (dark blue) were quantified after infection of MKN-28 cells with *Hp* for the indicated time periods. The data are calculated as the NTF/E-Cad-FL ratio from at least three independent experiments and presented as the mean ± S.D. in pie charts.
   (**B**) Recombinant E-Cadherin was incubated with 5 ng/µl MMP-7 for 16 h and increasing concentrations of MMP inhibitor II (IC₅₀ = 30 nM) as indicated. Loss of the extracellular E-Cadherin domain was detected by Western blot analysis using an anti-E-Cadherin antibody directed against the extracellular domain.
   (**C**) MKN-28 cells were infected with *Hp* or left untreated for indicated time periods. mRNA was isolated and transcribed into cDNA. Amplification of *mmp7, adam10* and *gapdh* was performed by PCR using specific primers.

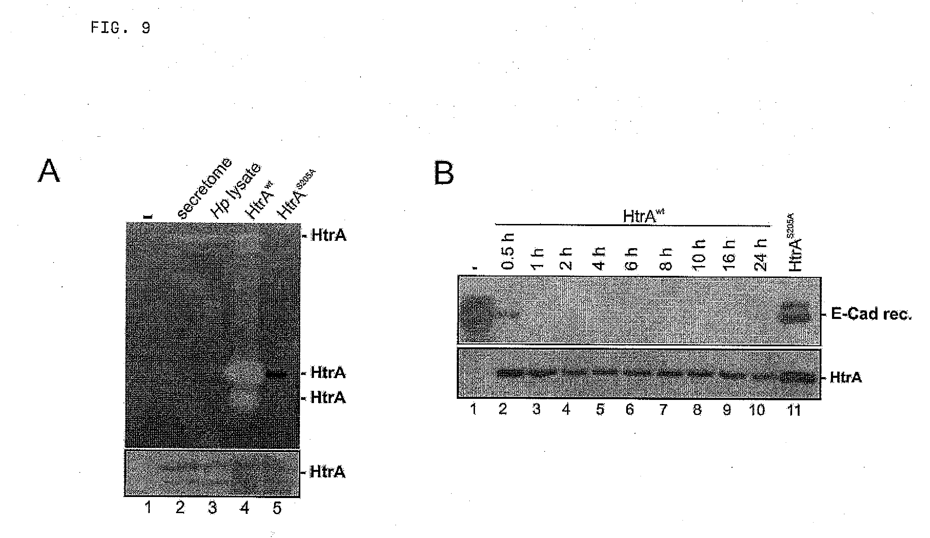

Figure 9: Specific cleavage of E-Cadherin by HtrA
   (**A**) *Hp* was grown in protein-free BHI medium. After 48 hours, the bacteria were harvested and lysed by sonication (*Hp* lysate). 30 µl aliquots of *Hp*-free BHI medium (lane 1), supernatants (secretome, lane 2), bacterial lysates (lane 3) and 3 µg of cloned and purified HtrA^{wt} or HtrA^{S2105A} (lanes 4, 5) were analyzed by casein zymography for proteolytic activity. Gels were also blotted on membranes and HtrA was detected using a specific antibody generated to detect HtrA.
   (**B**) 100 ng recombinant E-Cadherin (E-Cad rec.) were incubated with 100 ng/µl HtrA^{wt} for the indicated time periods. As a control inactive HtrA^{S205A} was used for 24 h. Loss of full length E-Cadherin and the presence of HtrA were analyzed in Western blots.

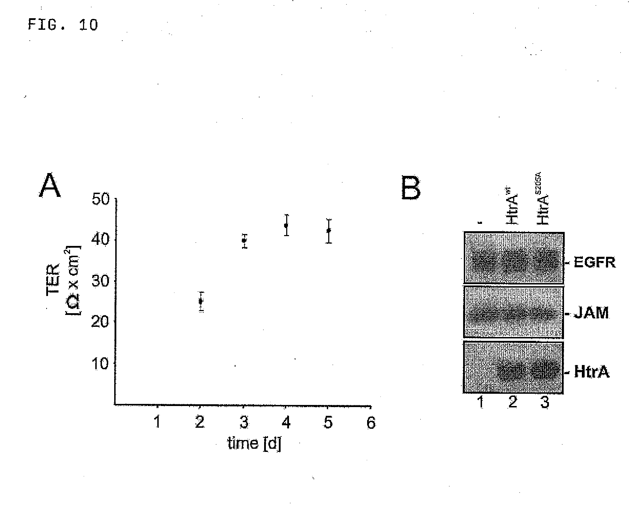

Figure 10: HtrA did not affect tight junctions
   (**A**) Formation of the MKN-28 cell monolayer was followed by transepithelial electrical resistance (TER) measuring for five days.
   (**B**) 100 ng of recombinant EGFR (epidermal growth factor) or JAM (junctional adhesion molecule) were incubated with 100 ng/µl of HtrA^{wt} or HtrA^{S205A}. EGFR, JAM and HtrA were detected by Western blots.

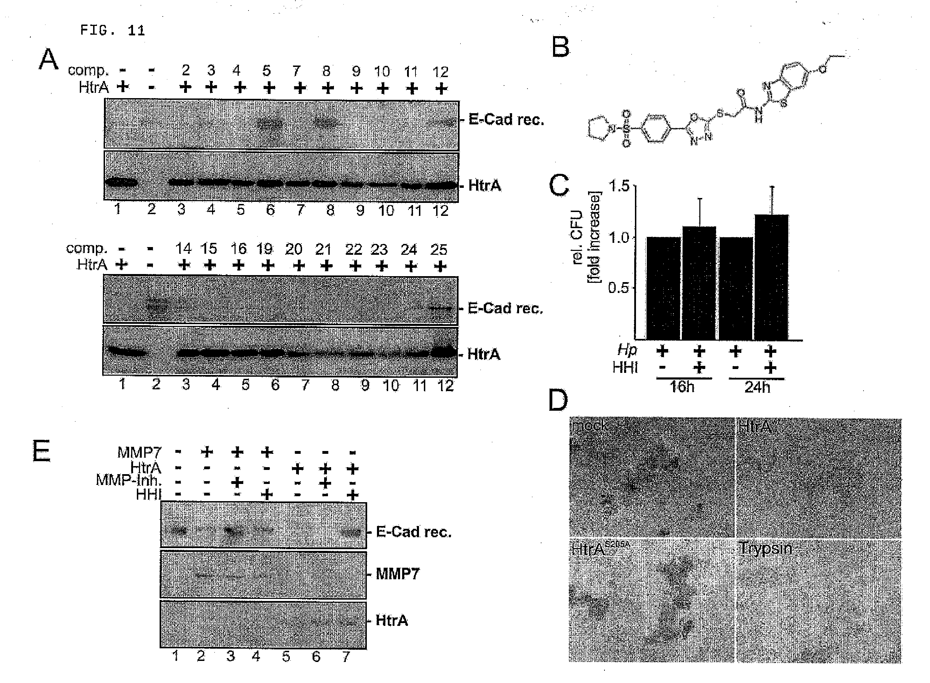

Figure 11: Inhibition of *Hp* HtrA by small molecule inhibitors
   (**A**) 100 ng recombinant E-Cadherin (E-Cad rec.) were incubated with 100 ng/µl HtrA together with 100 µM inhibitors as indicated for 2 h. E-Cadherin and HtrA were detected by Western blots.
   (**B**) Structure of the small inhibitor HHI (compound no. 8).
   (**C**) MKN-28 cells treated with 100 µM HHI or left untreated were infected with *Hp*. After 16 h and 24 h aliquots of cells scraped into PBS were cultured on *Hp* plates. *Hp* colonies counted after 72 h showed no significant difference in the relative CFU colony forming units (CFU).
   (**D**) E-Cadherin-mediated cell aggregation was investigated using untreated (mock), HtrA^{wt}, HtrA^{S205A} or trypsin-treated MCF-7 cells. Cell aggregates were observed using an inverse phase-contrast microscope.
   (**E**) 100 ng recombinant E-Cadherin (E-Cad rec.) were incubated with 5 ng/µl MMP-7 or 5 ng/µl HtrA for 16 h. As indicated MMP-inhibitor II (5 x IC₅₀) or 100 µM HHI were incubated with active proteases. Loss of the extracellular E-Cadherin domain, MMP-7 and HtrA were detected in Western blot analysis.

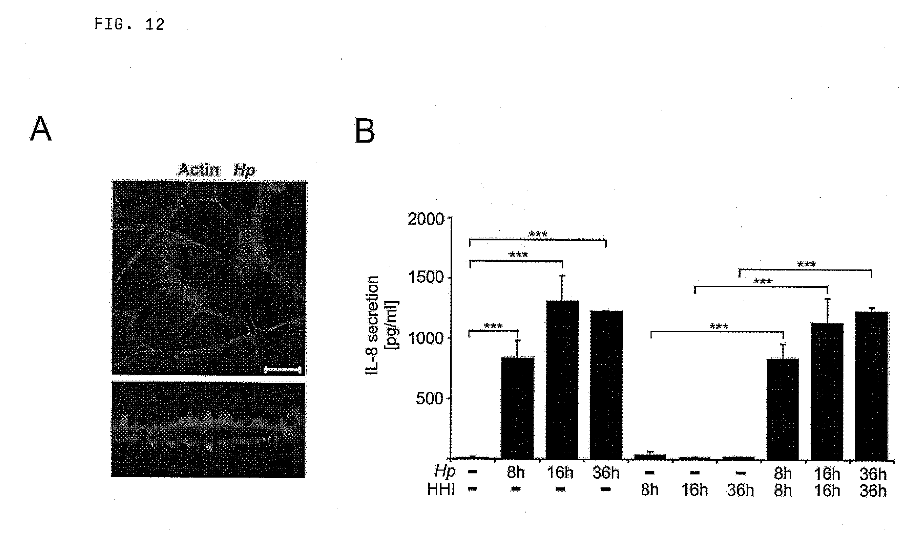

Figure 12: Functional T4SS-induced secretion of interleukin-8
   (**A**) MKN-28 cells were grown to confluence followed by infection with *Hp* at an MOI 100 for 24 h. Bacteria were stained using an anti-*Hp* antibody (green) and F-actin were counterstained using phalloidin (red) to demonstrate an intact monolayer.
   (**B**) Release of interleukin-8 (IL-8) was measured in ELISA assays using supernatant of untreated MKN-28 cells (-) and cell colonized with *Hp* for 8 h, 16 h and 36 h. Where indicated, MKN-28 cells were co-treated with 100 µM HHI. *** *p* < 0.001.

The present invention, thus, provides a method of screening for HtrA protease inhibitors useful for prophylaxis or therapy of a bacterial infection comprising the steps of
(a) providing a HtrA protease and a reporter protein cleavable by the HtrA protease;
(b) mixing the HtrA protease and the reporter protein in the presence of a suspected inhibitor of HtrA activity;
(c) detecting the enzymatic acivity of the HtrA protease in the presence of the suspected inhibitor; and
(d) comparing the HtrA activity in the presence of the suspected inhibitor to the activity in the absence of the inhibitor;
wherein said reporter protein is E-Cadherin or a recombinant reporter protein comprising the HtrA protease recognition sequence derived from E-Cadherin inserted in a permissive site of the reporter protein so as to retain biological activity of said reporter protein.

The screening method is carried out under conditions ensuring that the HtrA is biologically active and can interact with its substrate for cleavage. The method can be carried out in a cell-free system, a cell extract, a cell lysate or in a cell. The cell can be part of a cell culture or a non-human organism (animal model).

Preferably, the method of the present invention is a cell-based method, comprising the steps of
(a) co-expressing HtrA protease and a reporter protein cleavable by the HtrA protease in a host cell, wherein the cleavage of the reporter protein by the HtrA protease results in reduced activity of said reporter protein;
(b) exposing the host cell to suspected inhibitors of said HtrA protease; and
(c) screening for host cells that retain said reporter protein activity.

The term "exposing the host cell to suspected inhibitors of said HtrA protease" also includes the embodiment, wherein the inhibitor is a recombinant polypeptide or peptid that is encoded by a gene inserted into an expression vector that is present in the host cell.

Preferably, the cleavage of said reporter protein by the HtrA protease does not substantially result in accumulation of cleavage products capable of substantially inhibiting said protease.

The term "HtrA protease recognition sequence" refers to a consecutive amino acid sequence that is recognized by HtrA protease and is required for the proteolytic cleavage. A HtrA protease recognition sequence may be coincident with the protease cleavage site (i.e., the site at which the cleavage by the protease occurs). That is, the protease recognition sequence may include one or more amino acids on either side of the peptide bond to be hydrolyzed by the protease. Alternatively, the protease recognition sequence may be one, two or more amino acids distal, at the amino or carboxy terminus, to the cleavage site of the protease, as long as the cleavage of said reporter protein by said protease does not substantially result in accumulation of cleavage products capable of substantially inhibiting said protease.

The person skilled in the art is familiar with methods allowing expressing the proteins in sufficient amounts in the host cell such that potential inhibitors can be screened by inserting the genes encoding said proteins in suitable expression vectors. The expression vector comprises at least one nucleic acid sequence encoding a polypeptide or peptide selected from: a HtrA protease, a reporter protein and, optionally, a potential protease inhibitor, operably linked to one or more transcription control elements.

An "expression vector" refers to a nucleic acid molecule capable of replication and expressing a gene of interest when transformed, transfected or transduced into a host cell. The expression vectors comprise one or more phenotypic selectable markers and an origin of replication to ensure maintenance of the vector and to, if desired, provide amplification within the host. Selectable markers include, for example, sequences conferring antibiotic resistance markers, which may be used to obtain successful transformants by selection, such as ampicillin, tetracycline and kanamycin resistance sequences, or supply critical nutrients not available from complex media. Suitable expression vectors may be plasmids derived, for example, from pBR322 or various pUC plasmids, which are commercially available. Other expression vectors may be derived from bacteriophage, phagemid, or cosmid expression vectors, all of which are described in sections 1.12-1.20 of Sambrook et al., (Molecular Cloning: A Laboratory Manual. 3rd edn., 2001, Cold Spring Harbor Laboratory Press). Isolated plasmids and DNA fragments are cleaved, tailored, and ligated together in a specific order to generate the desired vectors, as is well known in the art (see, for example, Sambrook et al., ibid).

The test compounds (i.e. a suspected inhibitor) may be very different compounds, both naturally occurring compounds and synthetic, organic and inorganic compounds as well as polymers (e.g. oligopeptides, polypeptides, oligonucleotides and polynucleotides) as well as small molecules, antibodies, sugars, fatty acids, nucleotides and nucleotide analogs, analogs of naturally occurring structures (e.g. peptide "imitators", nucleic acid analogs, etc.) and numerous other compounds.

A large number of possibly useful test compounds can be screened in extracts of natural products as a starting material. Such extracts may be derived from a large number of sources, e.g. the following species: fungi, actinomycetes, algae, insects, protozoa, plants and bacteria. The extracts showing activity can then be analyzed for isolating the active molecule.

In a preferred embodiment of the screening method of the present invention, the test compound is from a library of fungal extracts. A particularly preferred library of fungal extracts is from a *Penicillium* or *Aspergillus* species.

The test compound may be a single compound or a plurality of compounds. Thus, test compounds can also be screened for on a large scale, e.g. by screening a very large number being able to contain synthetic or natural molecules. Thus, the test compound of a preferred embodiment of the method according to the invention forms part of a substance library. The term "plurality of compounds" in the screening method of the invention is to be understood as a plurality of substances which may or may not be identical. The plurality of compounds may be, e.g., added to the reaction mixture, culture medium, injected into a cell, preferably a mammalian cell, or otherwise applied to a non-human transgenic animal.

Commercially available libraries (e.g., from Affymetrix, ArQule, Neose Technologies, Sarco, Ciddco, Oxford Asymmetry, Maybridge, Aldrich, Panlabs, Pharmacopoeia, Sigma, or Tripose) can also be used with the new methods. In addition to libraries of potential inhibitors, special libraries called diversity files can be used to assess the specificity, reliability, or reproducibility of the screening methods of the invention. Diversity files contain a large number of compounds (e.g., 1000 or more small molecules) representative of many classes of compounds that could potentially result in non-specific detection in an assay. Diversity files are commercially available or can also be assembled from individual compounds commercially available from the vendors listed above.

If a sample containing a compound or a plurality of compounds is identified in the method of the invention, then it is either possible to isolate the compound from the original sample identified as containing the compound capable of suppressing protease activity or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the screening method of the invention only comprises a limited number of substances or only one substance. Preferably said sample comprises substances of similar chemical and/or physical properties, and most preferably said substances are identical.

In a particular preferred embodiment the inhibitor is 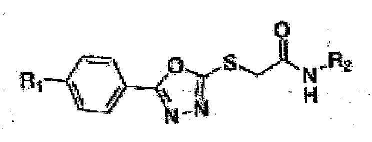 wherein:
R₁: hydrogen, hydroxyl, amide, sulfonamide, amine, unsubstituted or substituted heterocycle
R₂: hydrogen, aliphatic substituent, unsubstituted or substituted aromatic ring system, unsubstituted or substituted heterocycle.

The central ring system can be substituted by a tetrazole-based scaffold.

The above-mentioned expression "substituted" in connection R₁ and R₂ means that the following residues can independently be present singly, doubly or multiply, equally or unequally:
- OH, -SH, -O-C₁₋₈ alkyl, -O-C₆₋₁₄ aryl, -S-C₁₋₄ alkyl, -S-C₆₋₁₄ aryl,
- SO-C₁₋₄ alkyl, -SO-C₆₋₁₄ aryl, -SO₂-C₁₋₄ alkyl, -SO₂-C₆₋₁₄ aryl, - SO₃H,
- OSO₂C₁₋₈ alkyl, -OSO₂C₆₋₁₄ aryl, -COOH, -COOC₁₋₈ alkyl, -(CO)C₁₋₈ alkyl,
- COOH, -COOC₁₋₈ alkyl, -CONH₂, -CONHC₁₋₆ alkyl, -CON(C₁₋₆ alkyl)₂,
- NH₂, -NHC₁₋₆Alkyl, -N(C₁₋₆ alkyl)₂, -NHC₆₋₁₄ aryl, -NH- hetaryl,
- N(C₆₋₁₄ aryl)₂, -N(C₁₋₆ alkyl) (C₆₋₁₄ aryl),
- CH₃, -CHF₂, -CF₃, -C₂H₅, -C(CH₃)₂, -(CH₂) ₂CH₃, -(CH₂)₃CH₃,
- CH₂CH₂OH,
- CH₂CH₂SH, -CH₂CH₂SCH₃, -CH₂CF₃, -CH₂CCl₃, -CH₂CHF₂, -CH₂CHCl₂,
- CH₂CH₂F, -CH₂CH₂Cl, -CH₂CH₂Br, -cyclopropyl, - cyclopropylmethyl,
- cyclobutyl, -cyclobutylmethyl, -cyclopentyl, - cyclopentylmethyl,
- cyclohexyl, -cyclohexylmethyl,
- F, -Cl, -Br, -I, -CN, -NO₂, and -SCN.

"Aliphatic substituent" means any straight or branched C-atoms containing chain, preferably C1 - C14. The aliphatic substituent may an alkane, alkene or alkine residue. Preferred alkyl residues are methyl, ethyl, n-propyl, iso-propyl, n-butyl, tert-butyl, n-pentanyl, n-hexanyl, etc. The term "alkyl-", "alkenyl-" or "alkinyl", also in word combinations such as alk(en/in)ylsulfonyl, alk(en/in)ylamino or alk(en/in)oxycarbonyl etc., mean both unbranched and branched possible compounds. The aliphatic residue may also be substituted with functional groups as mentioned above, e.g. with -OH (-> alcohol), -CHO (-> aldehyde) or -COOH (-> carboxylic acid).

"Aromatic ring system" refers to an aromatic monocyclic or polycyclic ring system having 6 to 14 carbon atoms, preferably 6 to 10 carbon atoms. The ring system can be substituted, where appropriate, with one or several ring substituents as defined above. Preferred aromates are phenyl or naphthyl.

"Heterocycle" means monocyclic or bicyclic, saturated or monounsaturated or polyunsaturated heterocycles having 5 - 14 ring atoms, among them 1 - 5 heteroatoms which are preferably N, O and S: thienyl, pyridinyl, pyrimidinyl, piperazinyl, pyridyl, isoxazolyl, piperidinyl, pyrazinyl, morpholino, pyrrolyl, triazinyl, tetrazolyl, oxazolyl, benzo[d][1,3]dioxolyl, indolyl, imidazolyl, pyrazolyl, furanyl.

In a more preferred embodiment the inhibitor is

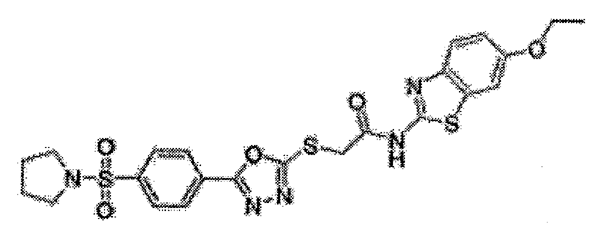

Host cells expressing the reporter protein are exposed to the HtrA protease and to potential inhibitors of the protease. Next, the host cell population is screened for cells that retain reporter protein activity. The term "exposed" is used herein to indicate that a host cell is contacted with a potential protease inhibitor such that the inhibitor can effectively inhibit the protease. Potential inhibitors may be expressed by the host cell, either naturally or upon the transformation of suitable constructs encoding them, or may be introduced externally, e.g. by addition of potential inhibitors to the culture medium. A potential inhibitor is identified as an inhibitor of the protease if the reporter protein is not cleaved and remains active when monitored by means of, for example, color change, light change, molecular weight/size change or other known methods of monitoring the activity. Host cells exposed to a functional inhibitor can thus be distinguished from host cells treated with an uninhibited protease, and are considered to "retain reporter protein activity".

Fundamentally suited assay formats for identifying positive test compounds are well known in the biotechnological and pharmaceutical industries and additional assays and variations of the above assays provided for the purpose of illustration are obvious to the person skilled in the art. The person skilled in the art can, e.g., draw on the assay formats given in the examples, below.

Compounds isolated by the screening method of the invention are suitable as therapeutics to treat, prevent, control, or lessen the severity of a bacterial infection, or can be used as lead compounds for the development of analog compounds, i.e. drugs that have additional desired properties, e.g. are capable of efficiently passing the blood-brain barrier. Moreover, such drugs should have a stabilized electronic configuration and molecular conformation that allows key functional groups to be presented in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said analogues can be tested for their effects according to methods known in the art; see also supra. Furthermore, peptidomimetics and/or computer aided design of appropriate derivatives and analogues can be used according to routine methods.

In a preferred embodiment of the screening method of the invention, (a) cleavage of the recombinant reporter protein generates a detectable signal or (b) the uncleaved reporter protein generates a detectable signal which substantially disappears after cleavage. Any reporter protein can be used in the screening method of the present invention, as long as: (i) the reporter protein is capable of being assayed for a change in color, light or activity when combined with the protease enzyme in a host cell; (ii) the introduction of the protease recognition site does not eliminate the activity of the reporter protein, so that the fusion protein can be used in an assay system to screen for inhibitors of said protease; and (iii) the cleavage of said reporter protein by said protease does not substantially result in accumulation of cleavage products capable of substantially inhibiting said protease.

When the protease enzyme is contacted with the resulting reporter protein, the protease cleaves at the cleavage site, thereby resulting in reduced activity of said reporter protein. The phrases "reduced activity of a reporter protein" and "substantially inhibiting a protease" refer to a detectable reduction in the activity of the reporter protein monitored by means of, for example, change in molecular weight/size, color change, light change or other known methods of monitoring the enzymatic activity. When determining whether the cleavage of a reporter protein by a HtrA protease substantially results in the accumulation of cleavage products capable of substantially inhibiting the protease, in addition to the functional assays for protease activity described above, lysates of host cells in which the reporter protein (and the protease are) expressed may be assayed for degradation products of the reporter protein. This may be achieved, for example, using antibodies recognizing the reporter protein, by methods well known in the art.

Examples for other reporters that may be utilized to generate a recombinant reporter protein of the invention include: alkaline phosphatase, beta-galactosidase, beta-glucuronidase, acetyltransferase, luciferase, green fluorescent protein (GFP), red fluorescent protein (RFP), an autofluorescent protein, including blue fluorescent protein (BFP), aequorin, chloramphenicol acetyl transferase, glutathiane-S-transferase (GST) and horseradish peroxidase.

In a more preferred embodiment of the screening method of the present invention the recombinant reporter protein comprises a galactosidase derivative and a HtrA protease recognition sequence derived from E-Cadherin inserted in a permissive site of β-galactosidase so as to retain β-galactosidase activity.

The term "β-galactosidase activity" as used herein indicates the ability of the reporter protein to hydrolyze lactose yielding glucose and galactose, allowing utilization of lactose as a carbon source. The enzymatic activity of β-galactosidase may be detected by utilizing suitable color indicator compounds, including, but not limited to X-gal and Fast Blue RR, which, upon their cleavage by β-galactosidase, produce a detectable color. Thus, for example, bacteria expressing said reporter protein appear as blue colonies when grown on a semi-solid medium-containing X-gal. Other substrates that may be used for detecting β-galactosidase activity include substrates that upon cleavage result in a fluorescent signal, e.g. Fluorescein-β-D-Galactopyranoside (FDG; commercially available from Marker Gene Technologies, Inc.), or in signals measurable by amperometric sensors with substrates such as PAPG.

In a preferred embodiment, the host cell is a bacterial host cell. In another preferred embodiment, the host cell is *E. coli.* Other suitable prokaryotic hosts include, but are not limited to: *Bacillus subtilis, Salmonella typhimurium* and various species within the genera *Pseudomonas, Streptomyces,* and *Staphylococcus.* In other embodiments, eukaryotic host cells are used, including, but not limited to plant cells, yeast cells, insect cells or animal cells, with suitable vectors and expression control elements known in the art.

According to certain embodiments of the screening method of the present invention, various methods may optionally be used to enrich for protease inhibitors prior to the screening step. The enrichment can be preformed before or after exposing the host cells to the potential protease inhibitors. For instance, when the inhibitors are expressed within the host cells, the host cell population may be enriched for inhibitor expressing clones. A non-limitative approach is described herein, in which a β-galactosidase derivative is used as a reporter protein in *E. coli.* Upon inducing the expression of the potential protease inhibitors, the cells are initially grown on lactose as a sole carbon source; the enrichment is brought about by virtue of faster growth rate of protease-inhibited clones. Other examples may include enriching a library for protease binders in vitro, e.g. by affinity selection. Screening for protease inhibitors is performed as described above, by identifying host cells which retain protease activity measured using e.g. color indicator compounds or light emitting reactions.

Cleavage of the reporter protein is also detectable by use of an antibody specifically binding to an epitope of the reporter protein comprising the recognition sequence that is destroyed by HtrA cleavage. The term "antibody" as used herein, preferably, relates to antibodies which consist essentially of pooled monoclonal antibodies with different epitopic specificities, as well as distinct monoclonal antibody preparations. Monoclonal antibodies are made from an antigen containing fragments of the Aβ peptides by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody.

The antibodies can be detectably labelled, for example, with a radioisotope, a bioluminescent compound, a chemiluminescent compound, a fluorescent compound, a metal chelate, or an enzyme (e.g. horse-radish peroxidase, alkaline phosphatase, beta-galactosidase, malate dehydrogenase, glucose oxidase, urease, catalase etc.) which, in turn, when later exposed to a substrate will react to the substrate in such a manner as to produce a chemical moiety which can be detected. The antibodies can also be immobilized on an insoluble carrier, e.g. glass, polystyrene, polypropylene, polyethylene, dextran, nylon, natural and modified celluloses, polyacrylamides, agarose and magnetic beads.

Any of a wide range of well known immunodetection techniques can be used. Examples of immunoassays suitable for the method of the present invention are competitive or sandwich assays, such as the enzyme linked immunosorbent assay (ELISA), the radioimmunoassay (RIA) or Western Blots. Suitable antibody assay labels are known in the art and include enzyme labels, such as, glucose oxidase, and radioisotopes, such as iodine (¹²⁵I, ¹²¹I), carbon (¹⁴C), sulphur (³⁵S), tritium (³H), indium (¹¹²In), and technetium (⁹⁹mTc), and fluorescent labels, such as fluorescein and rhodamine. Detection and/or quantification can also be carried out by MALDI-MS analysis.

The HtrA protease is obtainable from many microorganisms, e.g., *E.coli, S. aureus, Streptococcus pneumoniae, Streptococcus pyogenes, Helicobacter pylori* etc.. In the method of the present invention, HtrA protease obtained from *Helicobacter pylori* is preferred.

The present invention also provides an HtrA inhibitor, which is has the chemical structure depicted in Figure 11B. The inhibitor of the present invention can be administered to an organism per se, or in a pharmaceutical composition where it is mixed with suitable carriers or excipients. The purpose of a pharmaceutical composition is to facilitate administration of a compound to an organism.

Hereinafter, the phrases "physiologically acceptable carrier" and "pharmaceutically acceptable carrier", which may be used interchangeably, refer to a carrier or a diluent that does not cause significant irritation to an organism and does not abrogate the biological activity and properties of the administered inhibitor. Useful carriers include, for example, water, acetone, ethanol, ethylene glycol, propylene glycol, butane-1,3-diol, isopropyl myristate, isopropyl palmitate, or mineral oil.

Herein, the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils, and polyethylene glycols.

Techniques for formulation and administration of drugs may be found in the latest edition of "Remington' s Pharmaceutical Sciences," Mack Publishing Co., Easton, Pa., which is herein fully incorporated by reference.

Suitable routes of administration may, for example include oral, rectal, transmucosal, especially transnasal, intestinal, or parenteral delivery, including intramuscular, subcutaneous, and intramedullary injections, as well as intrathecal, direct intraventricular, intravenous, intraperitoneal or intranasal injections. Alternately, one may administer the pharmaceutical composition in a local rather than systemic manner, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes. Pharmaceutical compositions for use in accordance with the present invention, thus, may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen. For example, for injection, the active ingredients of the pharmaceutical composition may be formulated in aqueous solutions, preferably in physiologically compatible buffers such as Hank' s solution, Ringer's solution, or physiological salt buffer. For transmucosal administration, penetrants appropriate to the barrier to be permeated are used in the formulation. Such penetrants are generally known in the art. For oral administration, the pharmaceutical composition can be formulated readily by combining the HtrA inhibitor with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries as desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, and sodium carbomethylcellulose; and/or physiologically acceptable polymers such as polyvinylpyrrolidone (PVP).

Finally, the present invention also relates to the use of the HtrA inhibitor of the present invention for the preparation of a pharmaceutical composition for treating a bacterial infection, preferably a *Helicobacter pylori* infection.

The following examples illustrate the invention.

### Example 1

### General Methods

### (A) Cell culture

The human gastric adenocarcinoma cell line MKN-28 was grown in RPMI1640 medium (Biochrom, Berlin, Germany) and human breast cancer cells (MCF-7) in DMEM medium (Biochrom) containing 4 mM glutamine (Invitrogen, Karlsruhe, Germany]), and 10% FCS (Biowest, Nuaillé, France) in a humidified 5% CO₂ (MKN-28) or 10% CO₂ (MCF-7) atmosphere at 37°C. Cells were seeded in tissue culture plates 48 hours before infection and grown to confluence. 1-2 h hours prior to infection, medium was replaced by fresh serum-free media. Where indicated, cells were incubated with indicated concentrations of HHI or MMP inhibitor II (blocks MMP1, MMP3, MMP7 and MMP9) (Calbiochem, Merck , Darmstadt, Germany).

### (B) Bacteria and infection experiments

*Hp* strains Hp26695 and P12 were cultured on agar plates containing 10% horse serum under microaerophilic conditions at 37°C for 48 hours. For infection, bacteria were harvested in PBS Dulbecco's medium, pH 7.4, added to the host cells at a multiplicity of infection (MOI) of 50 or 100 for the indicated time periods and monitored using an inverse phase-contrast microscope (model TS 100, INTAS). To quantify bacteria translocating through cellular monolayers, MKN-28 cells were grown to confluence on filters prior to infection with *Hp* for 48 h. Translocating *Hp* were cultured and CFU were counted. All experiments were repeated 3-4 times.

For secretome analysis, *Hp* was grown in protein-free liquid brain heart infusion (BHI) medium (Merck) supplemented with β-cyclodextrin for 48 hours (Bumann et al., 2002). Supernatants of *Hp* BHI cultures were sterilized by filtration. Lysates of *Hp* were obtained by sonication in PBS containing 0.1% Triton X-100. To quantify colony forming units (CFU) of *Hp,* cells were infected with *Hp* in the presence or absence of HHI. After 16-24 h cells were washed twice with PBS, harvested in BHI supplemented with β-cyclodextrin and seeded on *Hp* plates. After 72 h, colonies were counted.

### (C) Immunoprecipitation, SDS-PAGE and Western blots

Cells were harvested in lysis buffer (20 mM Tris-HCl, pH 7.5, 1 mM ETDA, 100 mM NaCl, 1% Triton X-100, 0.5 % sodium deoxycholate, 0.1% SDS, 1 x complete protease inhibitors (Roche, Basel, Switzerland), 1 mM Na₃VO₄, 1 mM sodium molybdate, 20 mM NaF, 10 mM sodium pyrophosphate, 20 mM β-glycerophosphate) and separated by SDS-PAGE. Proteins were transferred onto PVDF membranes, blocked with RotiBlock (Roth, Karlsruhe, Germany), and probed with anti-E-Cadherin cl. 36 (detects intracellular E-Cadherin domain). To detect CTF1 and CTF2 by Western blots an anti-E-Cadherin polyclonal antibody from Cell Signaling Technologies , Danvers, MA, USA] was used. Quantification of CTF and NTF fragments was performed using the Fusion FX-7 image analyzer and Bio-1D analysis software (Vilber Lourmat Deutschland GmbH, Eberhardzell, Germany). Monoclonal antibodies HecD1 and H-108 against the extracellular domain of E-Cadherin (NTF) were obtained from Calbiochem, Merck, Darmstadt, Germany and Santa Cruz, Santa Cruz, CA, USA, respectively. Expression of Occludin, ZO-1 (Invitrogen), JAM1, β-Catenin, p120^{ctn} (BD Biosciences, New Jersey, USA), ADAM10, EGFR (Santa Cruz), GAPDH (Abcam, Cambridge, UK), β-Actin (Sigma), CagA (Aalto Bio Reagents Ltd., Dublin, Ireland) was analyzed using specific antibodies. Rabbit α - HtrA anti serum was raised against the amino-terminal peptide (C-DKIKVTIPGSNKEY) of *Hp* HtrA and was prepared by Biogenes GmbH (Berlin, Germany). Polyclonal antiserum (α-pCagA^{Y972}) was raised according to standard protocols (Biogenes) by immunization of two rabbits with a conserved CagA-derived phospho peptide (H-VGLSASPEPIpYAT, Jerini) conjugated to Limulus polyphemus haemocyanin carrier protein. The antibody was purified using affinity column and another column coupled with non-phospho CagA peptide in order to exclude recognition of the non-phosphorylated form of CagA. The specificity of the antibody for phosphorylated CagA was verified in a recent report (Kwok et al., 2007).

Interaction between β-Catenin and p120^{ctn} was analyzed by co-immunoprecipitation studies. Cells were lysed and β-Catenin and p120^{ctn} were immunoprecipitated from cell lysates. Immobilized protein complexes were washed three times with lysis buffer, boiled in Laemmli buffer, separated by SDS-PAGE, and blotted on membranes.

### (D) Protease purification and in vitro cleavage experiments

Genes for putative *Hp* proteases *hp0657*Δ*sp* (Δaa 1-20), *hp1012Δsp* (Δaa 1-26), and *hp0506*Δ*sp* (Δaa 1-25) (Lower et al., 2008) have been amplified from the genomic DNA of *Hp* strain Hp26695 by standard PCR using the Pfx DNA polymerase (Invitrogen). The following primers were used: *hp0657*Δ*sp* for: 5'-AGG ATC CTT GAC ACA CCA AGA AAT CAA TCA-3', *hp0657*Δ*sp* rev: 5'-AGA ATT CTC ATT TGT CCT TCT TTT TAT TGC TCA CA-3', *hp1012*Δ*sp* for: 5'-AGG ATC CCA ATC TTA CTT ACC CAA ACA-3', *hp1012*Δ*sp* rev: 5'-AGA ATT CTT AAG GTT TCA AAA ACA CGG T-3', *hp0506*Δ*sp* for: 5'-GGA TCC CTT TTA AAA GCC GAT GGA AT-3', *hp506*Δ*sp* rev: 5'-GAA TTC TTA AAA ACC CTC TAA AAG AT-3'. The amplified BamH1/EcoR1 flanked PCR product was then ligated into the pGEM-T Easy plasmid (Promega, Madison, USA), subcloned into the pGEX-6P-1 plasmid DNA (GE Healthcare Life Sciences, Buckinghamshire, UK) and transformed in *E. coli* BL21. Cloning and purification of HtrA^{wt} and HtrA^{S205A} have been described previously (Lower et al., 2008). Briefly, Sepharose-coupled HtrA^{wt} and HtrA^{S205A} were cleaved with PreScission Protease (GE Healthcare Life Sciences) for 16 h at 4°C to remove the GST tag.

For *in vitro* cleavage studies, recombinant E-Cadherin, EGFR and JAM chimera, IgA or Casein, (R&D Systems, Wiesbaden, Germany) were incubated with HtrA^{wt}, HtrA^{S205A} or MMP7 in 50 mM HEPES (pH 7.4) at 37°C for 16 h. Where indicated, HHI or MMP inhibitor II were added. Reactions were stopped by boiling in Laemmli buffer, followed by 8% SDS-PAGE and Western blotting.

### (E) Immunofluorescence

For immunofluorescence staining, cells were grown for 24 h on glass coverslips, then fixed (4% paraformaldehyde in PBS), and permeabilized (0.2% Triton X-100 in PBS). Immunostaining was performed using anti-E-Cadherin (cl. 36 detects the intracellular domain, BD Biosciences), anti-E-Cadherin (HecD-1 detects the extracellular domain), ZO-1 (Invitrogen), anti-CagA (Immunological and Biochemical Testsystems GmbH, Reutlingen, Germany), anti-β1-integrin and anti-*Hp*. Secondary antibodies were labeled with Alexa-488 or Alexa-546 (Invitrogen). Filamentous actin was detected with Alexa Fluor 546-conjugated phalloidin (Invitrogen). For nuclei staining, 4',6-diamin-2-phenylin-dol-dihydrochloride (DAPI, Roche) was used as recommended by the manufacturer's instructions. Samples were analyzed by confocal laser scanning microscopy using a Zeiss LSM 510 Meta confocal microscope. Images were further processed using Corel Photopaint.

### (F) HtrA modeling and virtual screening

A comparative protein "homology model" of HtrA based on the homolog DegP from *Escherichia coli* was built from the template PDB structure 3cs0 (Krojer et al., 2008) using the Molecular Operating Environment MOE, version 2007.09 (Chemical Computing Group, Montreal, Canada). The method used for structure-based virtual screening has been described elsewhere in detail (Löwer et al., in preparation; Schüller et al., 2006). In brief, the program PocketPicker (Weisel et al., 2007) was used to predict potential substrate binding sites on the surface of the protein model. Potential interactions (lipophilic, hydrogen-bond donors and acceptors) with the substrate are projected into this defined space. These potential pharmacophore points are clustered and transformed into a correlation vector by the program LIQUID (Tanrikulu et al., 2007). This vector representation allows for alignment-free comparison of the structure-derived pharmacophore model with compounds from a molecule database. Ligand docking was conducted with GOLD version 3.2 using the following parameter setting: autoscale = 1, popsiz = auto, select_pressure = auto, n_islands = auto, maxops = auto, niche_siz = auto, pt_crosswt = auto, allele_mutatewt = auto, migratewt = auto, radius = 10, origin = 0 0 0, do_cavity = 1, floodfill_atom_no = 0, param_file = DEFAULT, set_ligand_atom_types = 1, set_protein_atom_types = 0, tordist_file = DEFAULT, save_lone_pairs = 1, fit_points_file = fit_pts.mol2, read_fitpts = 0, internal_ligand_h_bonds = 0, n_ligand_bumps = 0, flip_free_corners = 0, flip_amide_bonds = 0, flip_planar_n = 1 flip_ring_NRR flip_ring_NHR, flip_pyramidal_n = 0, rotate_carboxylic_oh = flip, use_tordist = 1, postprocess_bonds = 1, rotatable_bond_override_file = DEFAULT, early_termination = 1, n_top_solutions = 3, rms_tolerance = 1.5, force_constraints = 0, covalent = 0, initial_virtual_pt_match_max = 3, relative_ligand_energy = 0, start_vdw_linear_cutoff = 6, score_param_file = DEFAULT. The following residues were defined as "active site residues": GLU207, LEU99, PHE182, SER241, ASN208, SER144, GLY244, VAL117, ILE201, HIS116, ASP147, TYR206, SER221, ILE253, MET257, ILE239, SER146, PRO218, PHE209, GLU145, GLY219, SER205, THR237, ALA238, GLY202, ASN217.

From the 26 top-ranking compounds selected for testing, six small molecule inhibitors efficiently blocked HtrA-mediated E-Cadherin cleavage *in vitro* (Fig. 11A). Compound 8 (HHI = *Hp* HtrA inhibitor; Fig. 11B) yielded a GoldScore of 61.47 indicating favorable binding, as confirmed by the cleavage assays (Fig. 11A).

### (G) Cell aggregation assay

For cell aggregation assays, 2 x 10⁵ cells were trypsinized and seeded into BSA-coated 24-well plates, each well containing 500 µl of cell aggregation buffer (10 mM HEPES (pH 7.4), 150 mM NaCl, 0.7 mM CaCl₂, 1% BSA, 0.01% trypsin). After stimulation at 37°C for 30 min on a rocking platform, cells were fixed in 500 µl of 4% paraformaldehyde for 20 min and observed using an inverse phase-contrast microscope.

### (H) Transepithelial electrical resistance (TER) assay

MKN-28 cells were cultured for five days on 0.33 cm² cell culture inserts with 0.4 µm pore size (Millipore) to form monolayers. TER was measured with an Electrical Resistance System (ERS) (Millipore, Bad Schwalbach/Taunus, Germany). Maximum resistance indicated cell confluence. TER was calculated as Ohms x cm² by subtracting fluid resistance and multiplying by the monolayer surface area.

### (I) Transfection, luciferase reporter gene experiments and siRNA

MKN-28 cells were cultured in 24-well plates and grown to 60% confluence. Cells were transfected with 100 ng TOPflash or 100 ng FOPflash (Upstate, Millipore, Bad Schwalbach/Taunus, Germany) and 4 ng phRL Null. As a positive control, cells were co-transfected with 100 ng β-Catenin (β-Cat^{S33A}) or empty vector for 12 h using Gene Juice transfection reagent (Novagen, Merck, Darmstadt, Germany) as recommended by the manufacturer's instructions. Eighteen hours after transfection, cells were infected with *Hp* at an MOI of 50, or left untreated. In further approaches, cells were incubated with 50 ng/µl HtrA^{wt} or HtrA^{S205A} mutant. After 24 h, cells were harvested in 100 µl reporter lysis buffer (Promega) and luciferase activity was determined in a dual channel luminometer. Results were normalized for transfection efficiency of cotransfected renilla luciferase plasmid.

For siRNA knock-down experiments, ADAM10 and control siRNA oligonucleotides (Santa Cruz Biotechnology) were transfected for 3 d according to the manufacturer's instructions.

### (J) Zymography

Zymographies have been previously described (Lower et al., 2008). Briefly, samples were loaded onto 8% SDS-PAGE containing 0.1% casein (Invitrogen) and separated by electrophoresis. After separation, the gel was renaturated in 2.5% Triton X-100, equilibrated in developing buffer (50 mM Tris-HCl, pH 7.4; 200 mM NaCl, 5 mM CaCl₂, 0.02% Brij35), and incubated overnight at 37°C in fresh developing buffer. Transparent bands of caseinolytic activity were visualized by staining with 0.5% Coomassie Blue R250.

### (K) RNA isolation and Polymerase Chain Reaction

Total RNA was isolated using the RNAeasy Midi Kit (Qiagen) as recommended by the manufacturer's instructions. Total RNA (1 µg) was reverse transcribed into single-stranded cDNA with Superscript II RT (Invitrogen) and oligo(dT) primers. For amplification following primer pairs were used: *mmp7* (for: 5'-AAA GAG ATC CCC CTG CAT TT-3', rev: 5'-GTG AGC ATC TCC TCC GAG AC-3') (Cury et al., 2007), *adam10* (for: 5'-AAT TCT GCT CCT CTC CTG GGC-3'; rev: 3'-TAT GTC CAG TGT AAA TAT GAG AGG-3') (Schirrmeister et al., 2009) and *gapdh* (for: 5'-TAT GAC AAC AGC CTC AAG AT-3', rev: 5'-AGG TCC ACC ACT GAC ACG TT-3'). PCR products were visualized by ethidium bromide staining after agarose gel electrophoresis.

### (L) Interleukin-8 (IL-8) Enzyme Linked Immunosorbent Assay (ELISA)

Determination of IL-8 release was performed by applying a commercially available IL-8 specific ELISA assay system (Bender Medsystems, eBioscience Inc., San Diegeo, CA, USA). In brief, MKN-28 cells were incubated with *Hp* 8-36 h. Cell culture supernatants were collected, centrifuged, and stored at -80°C for subsequent IL-8 quantification. The IL-8 release was determined as recommend by the manufacturer's instructions.

### (M) Statistical Analysis

All data were evaluated using Student t-test. P values = p < 0.05 were considered as statistically significant.

### Example 2

### Hp-secreted HtrA cleaves E-Cadherin

Full length 125 kDa E-Cadherin (E-Cad-FL) can be cleaved in the extracellular domain (NTF) by metalloproteases, generating a 40 kDa C-terminal fragment (CTF1), which can be further processed by γ-secretase-like activity into soluble 33 kDa CTF2 (Fig. 1A) (Marambaud et al., 2002). After colonizing gastric epithelial MKN-28 cells with *Hp*, we detected *Hp-*induced ectopic cleavage of E-Cadherin, as monitored by the extracellular NTF in cell supernatants. Conversely, E-Cad-FL amounts decreased in cell lysates, and CTF1, then CTF2 levels increased, as expected (Fig. 1B). Comparing NTF amounts to total E-Cad-FL in untreated and *Hp*-infected MKN-28 cells indicated that NTF supernatant levels increased from 0.3 ± 0.4% to 38.2 ± 11.8% after infection for 24 hours (Fig. 8A).

Cellular proteases such as MMP-7 and ADAM10 cleave E-Cadherin directly (Lochter et al., 1997; Marambaud et al., 2002; Maretzky et al., 2005), whose expression is actually induced upon *Hp* infection (Ogden et al., 2008; Schirrmeister et al., 2009). Hence, we tested whether MMPs or MADAM10 activities are involved in *Hp*-mediated E-Cadherin cleavage. Recombinant protein containing the extracellular E-Cadherin domain (E-Cad rec.) was degraded using recombinant MMP-7, which was inhibited by increasing concentrations of MMP Inhibitor II (Fig. 8B). In well agreement with previous studies (Ogden et al., 2008), MMP-7 was upregulated in *Hp*-infected MKN-28 cells (Fig. 8C). Independently of mRNA induction (Fig. 8C), we also observed an increase of mature ADAM10 protein in response to *Hp* which was strongly down-regulated using ADAM10 specific siRNA (Fig. 1C). However, neither inhibition of MMPs nor additional down-regulation of ADAM10 expression efficiently blocked *Hp*-mediated E-Cadherin cleavage on host cells (Fig. 1C) indicating that cellular host proteases play only a subordinate role in *Hp*-induced E-Cadherin shedding.

Previously, we observed that a secreted *Hp* factor induced ectopic E-Cadherin shedding (Weydig et al., 2007), which led us to hypothesize that a bacterial protease may cleave E-Cadherin. By comprehensive sequence screening of the *Hp* proteome for predicted secreted proteases, *Hp*-secreted HtrA was identified as an active caseinolytic protease with unknown functions (Lower et al., 2008). To analyze whether HtrA or one of the other protease candidates act as putative E-Cadherin proteases in an unbiased approach, HtrA, Hp0657, Hp1012 and Hp0506 were cloned and E-Cadherin cleavage, caseinase and IgAse activity was tested *in vitro* (Fig. 2A). As expected, HtrA degraded casein as an artificial substrate; while other tested putative *Hp* proteases were inactive (Fig. 2A). In fact, HtrA cleaved recombinant E-Cadherin *in vitro*, but not IgA (Fig. 2A) or EGFR and JAM (Fig. 10B) indicating that E-Cadherin represents the first biological substrate for *Hp-*secreted HtrA. To verify HtrA-mediated E-Cadherin cleavage, we cloned and purified wild-type HtrA (HtrA^{wt}) and an inactive variant (HtrA^{S205A}) (Lower et al., 2008), generated a specific HtrA antibody, and confirmed caseinase activity of purified HtrA^{wt}, as well as in the *Hp*-secreted protein fraction and bacterial cell lysate (Fig. 9A). To determine the activity of recombinant HtrA, recombinant E-Cadherin was incubated with increasing amounts of HtrA^{wt} or inactive HtrA^{S20SA}. Even low concentrations of HtrA^{wt}, but not HtrA^{S205A}, degraded E-Cadherin (Fig. 2B), while higher amounts effectively degraded E-Cadherin within 30 minutes (Fig. 9B).

HtrA also mediated E-Cadherin ectodomain release from polarized epithelial cells. MKN-28 cells were grown to confluence to form a monolayer followed by incubation with increasing amounts of purified HtrA. Only HtrA^{wt} increased soluble NTF in the supernatant and corresponding loss of E-Cad-FL (Fig. 2C). However, compared to concentrations added in *in vitro* experiments (Fig. 2B), the used concentration of 100 ng/µl HtrA was relatively high and might indicate inaccessible AJs in polarized cells. To investigate whether MKN-28 cells exhibiting accessible E-Cadherin on the cell surface respond to lower HtrA concentrations, detached MKN-28 cells were incubated with HtrA. In fact, low HtrA^{wt} concentrations were sufficient to cleave E-Cadherin as reflected by the loss of E-Cad-FL (Fig. 2D) supporting the hypothesis that *Hp*-secreted HtrA directly targets E-Cadherin on the surface of host cells.

In polarized epithelial cells TJs demarcate a border between apical and basolateral membranes. AJs are positioned below TJs exhibiting important tumor-suppressive and/or anti-metastasis properties (Wessler and Backert, 2008). Localization of ZO-1 and E-Cadherin in MKN-28 cells revealed the presence of TJs immediately above E-Cadherin-based AJs indicating polarization of gastric MKN-28 cells (Fig. 3A). To analyze how *Hp* overcomes the TJ barrier to cleave E-Cadherin in a confluent MKN-28 monolayer, the functionality of TJs was confirmed by determination of the transepithelial electrical resistance (TER) as described earlier (Wroblewski et al., 2009). When MKN-28 cells were grown to confluence, TER increased and reached a plateau implying impermeability of the monolayer (Fig. 10A). Upon infection with *Hp,* TER significantly decreased demonstrating loss of TJ function (Fig. 3B).

Occludin and JAM are key molecules in establishing and maintenance of TJs (Niessen, 2007); hence, it was investigated whether disruption of TJs occurs in response to HtrA, too.

Following an *Hp* infection over time, a rapid loss of occludin and JAM was observed (Fig. 3C) suggesting that these factors were either down-regulated or cleaved. Then it was analyzed if recombinant HtrA cleaves occludin or JAM on the host cell surface to loosen the intercellular adhesion. Neither occludin nor JAM were degraded after incubation with HtrA for 16 and 24 hours on MKN-28 cells (Fig. 3D) or *in vitro* (Fig. 10B). To further demonstrate the specificity of HtrA-mediated E-Cadherin cleavage EGFR (Fig. 10B) was also included in the study representing another target molecule in *Hp* infections (Keates et al., 2005).

### Example 3

### HHI represents a pharmacological lead structure that blocks HtrA

Generation of *htrA* knock-out mutants failed, confirming reports that HtrA is an essential *Hp* factor (Salama et al., 2004). We therefore focused on pharmacological inhibition and performed structure-based virtual screening for small molecule inhibitors against HtrA. A comparative protein model of HtrA (Fig. 4A) was used to create a pharmacophore model of the protease active site (Fig. 4B). After screening large compound databases, six small molecule inhibitors that efficiently blocked HtrA-mediated E-Cadherin cleavage *in vitro* (Fig. 11A) were identified. The selected inhibitor HHI (Fig. 11B) (*Hp* HtrA inhibitor; indicated as compound no. 8 in Fig. 11A) was docked into the active site of HtrA to determine a potential binding mode (Fig. 4B). When HtrA^{wt} was incubated with recombinant E-Cadherin together with increasing amounts of HHI, 10 µM was sufficient to partly block HtrA-mediated E-Cadherin cleavage, while an HHI concentration of 30 µM blocked cleavage completely (Fig. 4C).

In a biological context, *Hp*-induced ectopic E-Cadherin cleavage in infected MKN-28 cells was clearly inhibited in the presence of HHI (Fig. 4D). It could be excluded that this effect was due to HHI hindering secretion of HtrA into the infected cell supernatant (Fig. 4D), or interfering with *Hp* adherence and *Hp* viability (Fig. 11C). It could be further demonstrated that HHI did not affect MMP7-mediated E-Cadherin cleavage activity and that MMP inhibitor II did not block HtrA activity (Fig. 11E) demonstrating the high selectivity of HHI and MMP inhibitor II. In cell aggregation assays, MKN-28 cells no longer aggregated when cells were treated with trypsin or *Hp* lysate. In contrast to HtrA^{S205A}, HtrA^{wt}-treated cells also did not form aggregates, unless HHI was also added (Fig. 4E). Similar effects were observed in aggregation assays using another polarized cell line, MCF-7 (Fig. 11D), which was also used in an in-situ immunofluorescence assay to confirm *Hp-*induced loss of E-Cadherin-dependent intercellular adhesion and demonstrate that HHI blocks this *in-vivo* (Fig. 5A). Thus, HHI was identified to be an efficient lead structure for blocking HtrA-mediated disruption of intercellular adhesions.

### Example 4

### HHI prevents E-Cadherin cleavage, but not β-Catenin signaling

Since HHI efficiently blocked *Hp*-induced loss of E-Cadherin-mediated cell adhesion (Fig. 5A), it was further analyzed whether HtrA-mediated ectopic E-Cadherin shedding destabilizes the intracellular E-Cadherin protein complex composed of β-Catenin, p120 Catenin (p120^{ctn}) and α-Catenin, which exhibits important tumor-suppressive and/or anti-metastasis functions (Niessen, 2007). *Hp* disrupted the entire intracellular E-Cadherin protein complex as shown by immunoprecipitation of β-Catenin, whose interaction to p120^{ctn} via E-Cadherin was dissolved (Fig. 5B). Similar results were obtained in the reverse immunoprecipitation experiments. Upon *Hp* infection, a loss of β-Catenin in the precipitated p120^{ctn} immunocomplex was observed, which was not reverted by adding HHI (Fig. 5B). Free cytoplasmic β-Catenin can translocate into the nucleus, where it transactivates TCF/LEF1 target genes via the *wnt* signaling pathway; a process which is strongly implicated in carcinogenesis. The hypothesis that HtrA-dependent E-Cadherin cleavage did not lead to the release of β-Catenin from the E-Cadherin complex was further supported by monitoring nuclear β-Catenin functions, which has been well documented in *Hp-*colonized cells (Franco et al., 2005; Murata-Kamiya et al., 2007; Suzuki et al., 2009). Analyzing β-Catenin-mediated target gene expression, MKN-28 cells were transfected with TOPflash or FOPflash constructs followed by infection with *Hp* or treatment with HtrA. Compared to the positive control, i.e. activated β-Catenin (β-Cat^{S33A}), *Hp* induced a slight, but significant luciferase activity, which was unaffected by HHI. In contrast to cells transfected with constitutively active β-Catenin, TCF/LEF1-dependent target gene transactivation was also not observed after treatment with HtrA^{wt} or HtrA^{S205A} as controls (Fig. 5C). Taken together, the present data indicate that inhibition of HtrA-mediated E-Cadherin cleavage is not sufficient to stabilize the intracellular E-Cadherin complex. Hence, we conclude that intracellular CTF2 fragments (Fig. 1B), which are generated by intracellular proteases, may contribute to TCF/LEF1-dependent target gene expression and are involved in cell proliferation and differentiation in prolonged *Hp* infections.

### Example 5

### HtrA opens intercellular adhesions allowing CagA injection across β1-integrins

It was previously described that *Hp* injects the carcinogenic factor CagA using β1-integrins as basolateral receptors of the T4SS (Kwok et al., 2007). Indeed, *Hp* was seen to actively invade between cells of polarized MKN-28 monolayers when we stained for E-Cadherin (Fig. 6A), F-actin (Fig. 12A) and *Hp* (Figs. 6A, 12A) after 24-48 hours. *Hp* was clearly detected in intercellular spaces (Fig. 6A, white arrows), while HHI greatly reduced the number of translocating bacteria (Fig. 6A). HHI also significantly inhibited *Hp* invasion through the intercellular space of confluent MKN-28 cells growing on filters (Fig. 6B), while not affecting the adherence of *Hp* to the apical membranes of MKN-28 cells (Fig. 11C).

To investigate whether the HtrA-induced loss of E-Cadherin-mediated cell adhesion supports CagA injection into host cells, CagA phosphorylation was analyzed using an antibody recognizing phosphorylated CagA at tyrosine residue 972 also known as EPIYA-motif C (Kwok et al., 2007) in infected MKN-28 monolayers. Injected phospho-CagA was detected in *Hp*-infected cells, which decreased after HHI preincubation. Notably, the amount of total CagA was not altered (Fig. 7A). Demonstrating a functional T4SS, the T4SS-dependent secretion of interleukin-8 (IL-8) of *Hp*-infected cells was analyzed, which was unaffected by HHI (Fig. 12B) indicating specific action of HtrA on host cells. To analyze CagA translocation across β1-integrins, β1-integrin and CagA were visualized by confocal microscopy. After 24 hours infection, non-phospho CagA was found at the apical and basolateral site showing little co-localization with β1-integrin (Fig. 7B). However, detection of phospho-CagA revealed strong co-localization with β1-integrin at the basolateral membrane, which was efficiently prevented by HHI (Fig. 7C). These data indicate that CagA is injected across basolaterally expressed β1-integrins after HtrA-mediated E-Cadherin cleavage.

### Conclusion

### (A) Hp actively disrupts the epithelial barrier and cell polarity

Stable intercellular adhesion complexes are essential for the maintenance of epithelial integrity, which forms effective first barriers against pathogens, but also exhibits responsibilities in the development and progression of many diseases. One of the major proteins involved in maintaining epithelial cell-cell adhesion is E-Cadherin, a member of the Cadherin family of transmembrane adhesion proteins, which is involved in many cellular processes including morphogenesis, adhesion, recognition, communication and oncogenesis (Niessen, 2007). Here, the first evidence is provided that bacterial HtrA acts as a secreted protease that cleaves off E-Cadherin from gastric epithelial cells, enabling *Hp* to enter the intercellular space of polarized cells in a monolayer where it injects the pathogenic factor CagA across β1-integrins.

Intercellular adhesions in polarized monolayers are composed of TJs and AJs, which are actively disrupted by *Hp* (Mimuro et al., 2008; Hatakeyama, 2008). Several modes of action have been proposed how *Hp* targets AJs and TJs, involving injected CagA or soluble *Hp* factors (Amieva et al., 2003; Lytton et al., 2005; Saadat et al., 2007). Discrepancies could be explained by using either subconfluent or confluent monolayers, which develop functional intercellular adhesions. However, it was found that HtrA selectively affected E-Cadherin-based AJs in monolayers of polarized epithelial cells, but not TJs indicating coordinated, multiple steps in *Hp* pathogenesis. *Hp* induced a decrease of the TJ components occludin and JAM, which occurred independently of HtrA. This correlates well with earlier studies demonstrating that soluble *Hp* factors targets TJ functions, such as urease or VacA (Lytton et al., 2005; Papini et al., 1998; Wroblewski et al., 2009). From the present experiments, it is suggested that *Hp* secretes HtrA in its immediate environs to disrupt AJs allowing *Hp* to pass the epithelial layer and to contact β1-integrins. Injected CagA then activates cancer-associated signal transduction pathways which finally completely disrupt the epithelial polarity and integrity (Hatakeyama, 2008; Mimuro et al., 2008). Hence, the identification of HtrA-mediated cleavage of E-Cadherin is a crucial novel piece in the puzzle of *Hp* pathogenesis.

### (B) E-Cadherin cleavage is directly mediated by bacterial HtrA

Shedding of the E-Cadherin ectodomain occurs frequently and MMPs and ADAM10 have been described as responsible proteases (Lochter et al., 1997; Marambaud et al., 2002; Maretzky et al., 2005). Several MMP inhibitors which block a wide range of different MMPs (data not shown) were also used in the present studies to verify the hypothesis of a MMP-independent E-Cadherin cleavage. In addition to MMP inhibition ADAM10, which has been recently demonstrated as an E-Cadherin protease in *Hp*-infected cells (Schirrmeister et al., 2009) was also down-regulated. Only a slight reduction of *Hp*-mediated NTF was reached indicating that another protease plays a crucial role in *Hp*-induced E-Cadherin fragmentation.

The question arised whether a bacterial protease can cleave host cell proteins. The *Hp* collagenase Hp0169 was identified as the first active *Hp* protease that degrades type 1 collagen and represents an essential factor for colonization (Kavermann et al., 2003). The *Hp* genome was screened for putative exported proteases and among potential candidates, HtrA was the only active caseinolytic serine protease secreted by *Hp* (Lower et al., 2008). However, significant biological substrates for HtrA in *Hp* or other bacteria were completely unknown. The identification of *Hp*-secreted HtrA as an E-Cadherin protease is an important finding because it explains for the first time how *Hp* can invade an intact epithelium supporting early electron microscopy analyses of biopsies from cancer patients, where *Hp* was found in significant numbers in the intercellular space and at the basal lamina (Necchi et al., 2007). It has recently been described that *Hp*-mediated changes in cell polarity may be important for efficient *Hp* replication on host cells (Tan et al., 2009). Therefore, HtrA-mediated E-Cadherin cleavage contributes to the destruction of cell polarity and epithelial barrier function as an important mechanism in persistent *Hp* colonization and subsequently pathogenesis.

Ectopic E-Cadherin cleavage is apparently an important step in the pathogenesis of inflammatory responses or neoplastic transformation. Interestingly, one prognostic marker in gastric cancer is the frequent proteolytic release of the E-Cadherin ectodomain, which leads to increased cell invasion (Chan, 2006). Corresponding to MMP-inhibited cell aggregation (Noe et al., 2001), HtrA also blocked aggregation of MKN-28 cells demonstrating the importance the E-Cadherin NTF in adhesion of MKN-28 cells. It is further hypothesized that the NTF of E-Cadherin may function as a pseudo-ligand that blocks normal E-Cadherin interactions, resulting in reduced adhesion. Soluble E-Cadherin has been shown to upregulate MMP expression concomitantly with the induction of cell invasion in tumor cells (Nawrocki-Raby et al., 2003). Hence, it is tempting to speculate whether HtrA-mediated NTF in supernatants contributes to MMP induction observed after infection with *Hp*.

The present findings of a functional role of HtrA in *Hp* have important general implications of the HtrA protein family in other bacterial pathogens. Interestingly, HtrA is widely expressed among different gram-negative bacterial pathogens exhibiting important roles in virulence (Pallen and Wren, 1997).

### List of references

Amieva,M.R., Vogelmann,R., Covacci,A., Tompkins,L.S., Nelson, W.J., and Falkow,S. (2003). Disruption of the epithelial apical-junctional complex by Helicobacter pylori CagA. Science 300, 1430-1434.
Bumann,D., Aksu,S., Wendland,M., Janek,K., Zimny-Arndt,U., Sabarth,N., Meyer,T.F., and Jungblut,P.R. (2002). Proteome analysis of secreted proteins of the gastric pathogen Helicobacter pylori. Infect. Immun. 70, 3396-3403.
Chan,A.O. (2006). E-cadherin in gastric cancer. World J. Gastroenterol. 12, 199-203.
Clausen,T., Southan,C., and Ehrmann,M. (2002). The HtrA family of proteases: implications for protein composition and cell fate. Mol. Cell 10, 443-455.
Cury,P.R., de Araujo,V.C., Canavez,F., Furuse,C., Leite,K.R., and de Araujo,N.S. (2007). The effect of epidermal growth factor on matrix metalloproteinases and tissue inhibitors of metalloproteinase gene expression in cultured human gingival fibroblasts. Arch. Oral Biol. 52, 585-590.
Franco,A.T., Israel,D.A., Washington,M.K., Krishna,U., Fox,J.G., Rogers,A.B., Neish,A.S., Collier-Hyams,L., Perez-Perez,G.I., Hatakeyama,M., Whitehead,R., Gaus,K., O'Brien, D.P., Romero-Gallo,J., and Peek,R.M., Jr. (2005). Activation of beta-catenin by carcinogenic Helicobacter pylori. Proc. Natl. Acad. Sci. U. S. A 102, 10646-10651.
Fujikawa,A., Shirasaka,D., Yamamoto,S., Ota,H., Yahiro,K., Fukada,M., Shintani,T., Wada,A., Aoyama,N., Hirayama,T., Fukamachi,H., and Noda,M. (2003). Mice deficient in protein tyrosine phosphatase receptor type Z are resistant to gastric ulcer induction by VacA of Helicobacter pylori. Nat. Genet. 33, 375-381.
Hatakeyama,M. (2008). Linking epithelial polarity and carcinogenesis by multitasking Helicobacter pylori virulence factor CagA. Oncogene 27, 7047-7054.
Kavermann,H., Burns,B.P., Angermuller,K., Odenbreit,S., Fischer,W., Melchers,K., and Haas,R. (2003). Identification and characterization of Helicobacter pylori genes essential for gastric colonization. J. Exp. Med. 197, 813-822.
Keates,S., Keates,A.C., Nath,S., Peek,R.M., Jr., and Kelly,C.P. (2005). Transactivation of the epidermal growth factor receptor by cag+ Helicobacter pylori induces upregulation of the early growth response gene Egr-1 in gastric epithelial cells. Gut 54, 1363-1369.
Krojer,T., Sawa,J., Schafer,E., Saibil,H.R., Ehrmann,M., and Clausen,T. (2008). Structural basis for the regulated protease and chaperone function of DegP. Nature 453, 885-890.
Kwok,T., Zabler,D., Urman,S., Rohde,M., Hartig,R., Wessler,S., Misselwitz,R., Berger,J., Sewald,N., Konig,W., and Backert,S. (2007). Helicobacter exploits integrin for type IV secretion and kinase activation. Nature 449, 862-866.
Lochter,A., Galosy,S., Muschler,J., Freedman,N., Werb,Z., and Bissell,M.J. (1997). Matrix Metalloproteinase Stromelysin-1 Triggers a Cascade of Molecular Alterations That Leads to Stable Epithelial-to-Mesenchymal Conversion and a Premalignant Phenotype in Mammary Epithelial Cells. J. Cell Biol. 139, 1861-1872.
Lower,M., Weydig,C., Metzler,D., Reuter,A., Starzinski-Powitz,A., Wessler,S., and Schneider,G. (2008). Prediction of extracellular proteases of the human pathogen Helicobacter pylori reveals proteolytic activity of the Hp1018/19 protein HtrA. PLoS. ONE. 3, e3510.
Löwer,M., Weydig,C., Wessler,S., & Schneider,G. Inhibitors of Helicobacter pylori protease HtrA found by "virtual ligand" screening block E-cadherin cleavage in vitro and combat bacterial infection. (2010). In preparation*.*
Lytton,S.D., Fischer,W., Nagel,W., Haas,R., and Beck,F.X. (2005). Production of ammonium by Helicobacter pylori mediates occludin processing and disruption of tight junctions in Caco-2 cells. Microbiology 151, 3267-3276.
Marambaud,P., Shioi,J., Serban,G., Georgakopoulos,A., Sarner,S., Nagy,V., Baki,L., Wen,P., Efthimiopoulos,S., Shao,Z., Wisniewski,T., and Robakis,N.K. (2002). A presenilin-1/gamma-secretase cleavage releases the E-cadherin intracellular domain and regulates disassembly of adherens junctions. EMBO J. 21, 1948-1956.
Maretzky,T., Reiss,K., Ludwig,A., Buchholz,J., Scholz,F., Proksch,E., de Strooper,B., Hartmann,D., and Saftig,P. (2005). ADAM10 mediates E-cadherin shedding and regulates epithelial cell-cell adhesion, migration, and beta-catenin translocation. Proc. Natl. Acad. Sci. U. S. A 102, 9182-9187.
Matsumoto,Y., Marusawa,H., Kinoshita,K., Endo,Y., Kou,T., Morisawa,T., Azuma,T., Okazaki,I.M., Honjo,T., and Chiba,T. (2007). Helicobacter pylori infection triggers aberrant expression of activation-induced cytidine deaminase in gastric epithelium. Nat. Med. 13, 470-476.
Mimuro,H., Berg,D.E., and Sasakawa,C. (2008). Control of epithelial cell structure and developmental fate: lessons from Helicobacter pylori. Bioessays 30, 515-520.
Murata-Kamiya,N., Kurashima,Y., Teishikata,Y., Yamahashi,Y., Saito,Y., Higashi,H., Aburatani,H., Akiyama,T., Peek,R.M., Jr., Azuma,T., and Hatakeyama,M. (2007). Helicobacter pylori CagA interacts with E-cadherin and deregulates the beta-catenin signal that promotes intestinal transdifferentiation in gastric epithelial cells. Oncogene 26, 4617-4626.
Nawrocki-Raby,B., Gilles,C., Polette,M., Bruyneel,E., Laronze,J.Y., Bonnet,N., Foidart,J.M., Mareel,M., and Birembaut,P. (2003). Upregulation of MMPs by soluble E-cadherin in human lung tumor cells. Int. J. Cancer 105, 790-795.
Necchi,V., Candusso,M.E., Tava,F., Luinetti,O., Ventura,U., Fiocca,R., Ricci,V., and Solcia,E. (2007). Intracellular, intercellular, and stromal invasion of gastric mucosa, preneoplastic lesions, and cancer by Helicobacter pylori. Gastroenterology 132, 1009-1023.
Niessen,C.M. (2007). Tight junctions/adherens junctions: basic structure and function. J. Invest Dermatol. 127, 2525-2532.
Noe,V., Fingleton,B., Jacobs,K., Crawford,H.C., Vermeulen,S., Steelant,W., Bruyneel,E., Matrisian,L.M., and Mareel,M. (2001). Release of an invasion promoter E-cadherin fragment by matrilysin and stromelysin-1. J. Cell Sci. 114, 111-118.
Ogden,S.R., Wroblewski,L.E., Weydig,C., Romero-Gallo,J., O'-Brien,D.P., Israel,D.A., Krishna,U.S., Fingleton,B., Reynolds,A.B., Wessler,S., and Peek,R.M., Jr. (2008). p120 and Kaiso regulate Helicobacter pylori-induced expression of matrix metalloproteinase-7. Mol. Biol. Cell 19, 4110-4121.
Pallen,M.J. and Wren,B.W. (1997). The HtrA family of serine proteases. Mol. Microbiol. 26, 209-221.
Papini,E., Satin,B., Norais,N., de,B.M., Telford,J.L., Rappuoli,R., and Montecucco,C. (1998). Selective increase of the permeability of polarized epithelial cell monolayers by Helicobacter pylori vacuolating toxin. J. Clin. Invest 102, 813-820.
Peek,R.M., Jr. and Crabtree,J.E. (2006). Helicobacter infection and gastric neoplasia. J. Pathol. 208, 233-248.
Saadat,I., Higashi,H., Obuse,C., Umeda,M., Murata-Kamiya,N., Saito,Y., Lu,H., Ohnishi,N., Azuma,T., Suzuki,A., Ohno,S., and Hatakeyama,M. (2007). Helicobacter pylori CagA targets PAR1/MARK kinase to disrupt epithelial cell polarity. Nature 447, 330-333.
Salama,N.R., Shepherd,B., and Falkow,S. (2004). Global transposon mutagenesis and essential gene analysis of Helicobacter pylori. J. Bacteriol. 186, 7926-7935.
Schirrmeister,W., Gnad,T., Wex,T., Higashiyama,S., Wolke,C., Naumann,M., and Lendeckel,U. (2009). Ectodomain shedding of E-cadherin and c-Met is induced by Helicobacter pylori infection. Exp. Cell Res. 315:3500-8.
Schuller,A., et al. (2006). A pseudo-ligand approach to virtual screening. Comb. Chem. High Throughput Screen. 9, 359.
Suzuki,M., Mimuro,H., Kiga,K., Fukumatsu,M., Ishijima,N., Morikawa,H., Nagai,S., Koyasu,S., Gilman,R.H., Kersulyte,D., Berg,D.E., and Sasakawa,C. (2009). Helicobacter pylori CagA phosphorylation-independent function in epithelial proliferation and inflammation. Cell Host. Microbe 5, 23-34.
Suzuki,M., Mimuro,H., Suzuki,T., Park,M., Yamamoto,T., and Sasakawa,C. (2005). Interaction of CagA with Crk plays an important role in Helicobacter pylori-induced loss of gastric epithelial cell adhesion. J. Exp. Med. 202, 1235-1247.
Tan,S., Tompkins,L.S., and Amieva,M.R. (2009). Helicobacter pylori usurps cell polarity to turn the cell surface into a replicative niche. PLoS. Pathog. 5, e1000407.
Tanrikulu,Y., Nietert,M., Scheffer,U., Proschak,E., Grabowski,K., Schneider,P., Weidlich,M., Karas,M., Gobel,M., and Schneider,G. (2007). Scaffold hopping by "fuzzy" pharmacophores and its application to RNA targets. Chembiochem. 8, 1932-1936.
Weisel,M., Proschak,E., and Schneider,G. (2007). PocketPicker: analysis of ligand binding-sites with shape descriptors. Chem. Cent. J. 1, 7.
Wessler,S. and Backert,S. (2008). Molecular mechanisms of epithelial-barrier disruption by Helicobacter pylori. Trends Microbiol. 16, 397-405.
Weydig,C., Starzinski-Powitz,A., Carra,G., Lower,J., and Wessler,S. (2007). CagA-independent disruption of adherence junction complexes involves E-cadherin shedding and implies multiple steps in Helicobacter pylori pathogenicity. Exp. Cell Res. 313, 3459-3471.
Wroblewski,L.E., Shen,L., Ogden,S., Romero-Gallo,J., Lapierre,L.A., Israel,D.A., Turner,J.R., and Peek,R.M., Jr. (2009). Helicobacter pylori dysregulation of gastric epithelial tight junctions by urease-mediated myosin II activation. Gastroenterology 136, 236-246.

## Claims

1. A method of screening for HtrA protease inhibitors useful for prophylaxis or therapy of a bacterial infection comprising the steps of
(a) providing an HtrA protease and a reporter protein cleavable by the HtrA protease;
(b) mixing the HtrA protease and the reporter protein in the presence of a suspected inhibitor of HtrA activity;
(c) detecting the enzymatic acivity of the HtrA protease in the presence of the suspected inhibitor; and
(d) comparing the HtrA activity in the presence of the suspected inhibitor to the activity in the absence of the inhibitor;
wherein said reporter protein is E-Cadherin or a recombinant reporter protein comprising the HtrA protease recognition sequence derived from E-Cadherin inserted in a permissive site of the reporter protein so as to retain biological activity of said reporter protein.

2. The method of claim 1, comprising the steps of
(a) co-expressing HtrA protease and a reporter protein cleavable by the HtrA protease in a host cell, wherein the cleavage of the reporter protein by the HtrA protease results introduced activity of said reporter protein;
(b) exposing the host cell to suspected inhibitors of said HtrA protease; and
(c) screening for host cells that retain said reporter protein activity.

3. The method of claim 1 or 2, wherein (a) cleavage of the recombinant reporter protein generates a detectable signal or (b) the uncleaved reporter protein generates a detectable signal which substantially disappears after cleavage.

4. The method of claim 3, wherein the recombinant reporter protein comprises a galactosidase derivative and a HtrA protease recognition sequence derived from E-Cadherin inserted in a permissive site of β-galactosidase so as to retain β-galactosidase activity.

5. The method of any one of claims 1 to 4, wherein the HtrA protease is a *Helicobacter pylori* protease.

6. The method of any one of claims 1 to 5, wherein said host cell is a bacterial host cell.

7. The method of claim 6, wherein the host cell is E. coli.

8. An inhibitor of a HtrA protease, which has the chemical formula 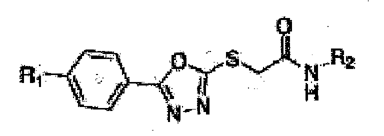 wherein:
R₁: hydrogen, hydroxyl, amide, sulfonamide, amine, unsubstituted or substituted heterocycle
R₂: hydrogen, aliphatic substituent, unsubstituted or substituted aromatic ring system, unsubstituted or substituted heterocycle.

9. The inhibitor of claim 8 having the formula:

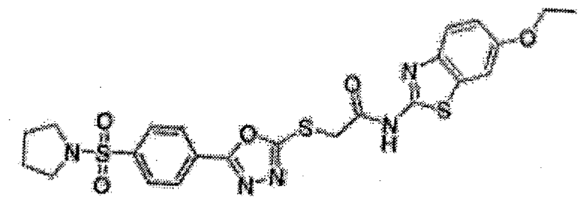

10. A pharmaceutical containing the inhibitor of claim 8 or 9.

11. The inhibitor of claim 8 or 9 for use in a method of treatment of a bacterial infection.

12. Use of the inhibitor of claim 8 or 9 for the preparation of a pharmaceutical composition for treating a bacterial infection.

13. Inhibitor of claim 8 or 9 , or use according to claim 12, wherein said bacterial infection is a *Helicobacter pylori* infection.


SEQUENCE LISTING

<110>  Paul-Ehrlich-Institut

<120>  Method of screening for HtrA protease inhibitors useful for
       prophylaxis and therapy of a bacterial infection

<130>  P 3234EP

<160>  14

<170>  PatentIn version 3.3

<210>  1
<211>  30
<212>  DNA
<213>  artificial sequence

<220>
<223>  primer hp0657deltasp

<400>  1
aggatccttg acacaccaag aaatcaatca                                        30


<210>  2
<211>  35
<212>  DNA
<213>  artificial sequence

<220>
<223>  primer hp0657deltasp rev

<400>  2
agaattctca tttgtccttc tttttattgc tcaca                                  35


<210>  3
<211>  27
<212>  DNA
<213>  artificial sequence

<220>
<223>  primer hp1012deltasp for

<400>  3
aggatcccaa tcttacttac ccaaaca                                           27


<210>  4
<211>  28
<212>  DNA
<213>  artificial sequence

<220>
<223>  primer hp1012deltasp rev

<400>  4
agaattctta aggtttcaaa aacacggt                                          28


<210>  5
<211>  26
<212>  DNA
<213>  artificial sequence

<220>
<223>  primer0506deltasp for

<400>  5
ggatcccttt taaaagccga tggaat                                          26


<210>  6
<211>  26
<212>  DNA
<213>  artificial sequence

<220>
<223>  primer hp506deltasep rev

<400>  6
gaattcttaa aaaccctcta aaagat                                          26


<210>  7
<211>  20
<212>  DNA
<213>  artificial sequence

<220>
<223>  primer mmp7 for

<400>  7
aaagagatcc ccctgcattt                                                 20


<210>  8
<211>  20
<212>  DNA
<213>  artificial sequence

<220>
<223>  primer mmp7 rev

<400>  8
gtgagcatct cctccgagac                                                 20


<210>  9
<211>  21
<212>  DNA
<213>  artificial sequence

<220>
<223>  primer adam10 for

<400>  9
aattctgctc ctctcctggg c                                               21


<210>  10
<211>  24
<212>  DNA
<213>  artificial sequence

<220>
<223>  primer adam10 rev

<400>  10
tatgtccagt gtaaatatga gagg                                            24


<210>  11
<211>  20
<212>  DNA
<213>  artificial sequence

<220>
<223>  primer gapdh for

<400>  11
tatgacaaca gcctcaagat                                                    20


<210>  12
<211>  20
<212>  DNA
<213>  artificial sequence

<220>
<223>  primer gapdh rev

<400>  12
aggtccacca ctgacacgtt                                                    20


<210>  13
<211>  14
<212>  PRT
<213>  artificial sequence

<220>
<223>  amino terminal peptide of HpHtrA

<400>  13

Asp Lys Ile Lys Val Thr Ile Pro Gly Ser Asn Lys Glu Tyr
1               5                   10


<210>  14
<211>  13
<212>  PRT
<213>  artificial sequence

<220>
<223>  CagA-derived phospho peptide


<220>
<221>  PHOSPHORYLATION
<222>  (11)..(11)

<400>  14

Val Gly Leu Ser Ala Ser Pro Glu Pro Ile Tyr Ala Thr
1               5                   10